# EUROPEAN PATENT SPECIFICATION

(11) **EP 1 320 611 B1**
(45) Date of publication and mention of the grant of the patent: **09.11.2005**
(21) Application number: 01973782.4
(22) Date of filing: 26.04.2001
(51) Int. Cl.: C12N 15/52, C12N 15/74, C12N 9/00, C12N 1/21, C12P 17/16, C12P 17/18, C07D 313/00, C07D 413/06, C07D 417/06, C07D 493/04, C07D 493/06

(54) **HETEROLOGOUS PRODUCTION OF POLYKETIDES**
HETEROLOGE HERSTELLUNG VON POLYKETIDEN
PRODUCTION HETEROLOGUE DE POLYKETIDES

(30) Priority: 28.04.2000 US 560367; 14.09.2000 US 232696 P; 21.12.2000 US 257517 P; 03.04.2001 US 825856; 03.04.2001 US 825876; 13.02.2001 US 269020 P
(43) Date of publication of application: 25.06.2003
(73) Proprietor: Kosan Biosciences, Inc., Hayward, CA 94545 (US)
(72) Inventor: ARSLANIAN, Robert, L., Pacifica, CA 94044 (US); ASHLEY, Gary, Alameda, CA 94502 (US); FRYKMAN, Scott, Hayward, CA 94544 (US); JULIEN, Bryan, Oakland, CA 94619 (US); KATZ, Leonard, Oakland, CA 94611 (US); KHOSLA, Chaitan, Palo Alto, CA 94306 (US); LAU, Janice, San Mateo, CA 94403 (US); LICARI, Peter, J., Fremont, CA 94536 (US); REGENTIN, Rika, Hayward, CA 94542 (US); SANTI, Daniel, San Francisco, CA 94117 (US); TANG, Li, Foster City, CA 94404 (US)
(74) Representative: Roques, Sarah Elizabeth
(86) International application number: PCT/US2001/013793
(87) International publication number: WO 2001/083800

(56) References cited:
- WO-A-00/22139
- WO-A-00/31247
- WO-A-98/22461
- WO-A-99/66028
- TANG L ET AL.: "Cloning and heterologous expression of the epothilone gene cluster" SCIENCE, vol. 287, no. 5453, 2000, pages 640-642, XP000910114 ISSN: 0036-8075
- BEYER S ET AL.: "Metabolic diversity in myxobacteria: identification of the myxalamid and the stigmatellin biosynthetic gene cluster of Stigmatella aurantiaca Sg a15 and a combined polyketide-(poly)peptide gene cluster from the epothilone producing strain Sorangium cellulosum So ce90" BIOCHIMICA ET BIOPHYSICA ACTA. GENE STRUCTURE AND EXPRESSION, vol. 1445, no. 2, 14 May 1999 (1999-05-14), pages 185-195, XP004275376 ISSN: 0167-4781
- SILAKOWSKI B ET AL.: "New lessons for combinatorial biosynthesis from myxobacteria: The myxothiazol biosynthetic gene cluster of Stigmatella aurantiaca DW4/3-1." JOURNAL OF BIOLOGICAL CHEMISTRY, vol. 274, no. 52, 24 December 1999 (1999-12-24), pages 37391-37399, XP002199229 ISSN: 0021-9258
- PAITAN Y ET AL.: "The first gene in the biosynthesis of the polyketide antibiotic TA of Myxococcus xanthus codes for a unique PKS module coupled to a peptide synthetase" JOURNAL OF MOLECULAR BIOLOGY, LONDON, GB, vol. 286, no. 1, 1999, pages 465-474, XP000920956 ISSN: 0022-2836
- SHIMKETS L J: "Industrial relevance and genetic analysis of myxobacteria." INDUSTRIAL MICROORGANISMS: BASIC AND APPLIED MOLECULAR GENETICS., 1993, pages 85-96, XP008003384 Fifth ASM;Bloomington, Indiana, USA; October 11-15, 1992, American Society for Microbiology (ASM) Books Division, 1325 Massachusetts Ave. NW, Washington, DC 20005-4171, USA ISBN: 1-55581-063-2
- NICOLAOU K C ET AL.: "Chemical Biology of Epothilones" ANGEWANDTE CHEMIE INTERNATIONAL EDITION, vol. 37, no. 15, August 1998 (1998-08), pages 2014-2045, XP002131418 ISSN: 0570-0833
- BRETSCHER A P ET AL.: "Nutrition of Myxococcus xanthus, a fruiting Myxobacterium" JOURNAL OF BACTERIOLOGY, vol. 133, no. 2, 1978, pages 763-768, XP001010255 ISSN: 0021-9193
- REGENTIN R ET AL.: "Development of a cost effective epothilone D process in Myxococcus xanthus." ABSTRACTS OF PAPERS AMERICAN CHEMICAL SOCIETY, vol. 221, no. 1-2, 1 April 2001 (2001-04-01), page BIOT 61 XP008003385 221st National Meeting of the American Chemical Society;San Diego, California, USA; April 01-05, 2001 ISSN: 0065-7727
- PFEIFER B A ET AL.: "Biosynthesis of polyketides in heterologous hosts" MICROBIOLOGY AND MOLECULAR BIOLOGY REVIEWS, vol. 65, no. 1, March 2001 (2001-03), pages 106-118, XP000997626 ISSN: 1092-2172
- FRYKMAN S ET AL.: "Modulation of epothilone analog production through media design." JOURNAL OF INDUSTRIAL MICROBIOLOGY & BIOTECHNOLOGY, vol. 28, no. 1, January 2002 (2002-01), pages 17-20, XP008003393 ISSN: 1367-5435
- ARSLANIAN R L ET AL.: "Large-scale isolation and crystallization of epothilone D from Myxococcus xanthus cultures." JOURNAL OF NATURAL PRODUCTS, vol. 65, no. 4, April 2002 (2002-04), pages 570-572, XP001074701 ISSN: 0163-3864
- LAU J ET AL.: "Optimizing the heterologous production of epothilone D in Myxococcus xanthus." BIOTECHNOLOGY AND BIOENGINEERING, vol. 78, no. 3, 5 May 2002 (2002-05-05), pages 280-288, XP002199234 ISSN: 0006-3592

## Description

### Field of the Invention

The present invention provides recombinant methods and materials for producing polyketides in recombinant host cells.

### Background of the Invention

Polyketides constitute a class of structurally diverse compounds synthesized, at least in part, from two carbon unit building block compounds through a series of Claisen type condensations and subsequent modifications. Polyketides include antibiotics such as tetracycline and erythromycin, anticancer agents such as the epothilones and daunomycin, and immunosuppressants such as FK506, FK520, and rapamycin. Polyketides occur naturally in many types of organisms, including fungi and mycelial bacteria. Polyketides are synthesized *in vivo* by polyketide synthase enzymes commonly referred to as PKS enzymes. Two major types of PKS are known that differ in their structure and the manner in which they synthesize polyketides. These two types are commonly referred to as Type I or modular and Type II or iterative (aromatic) PKS enzymes.

The present invention provides methods and recombinant expression vectors and host cells for the production of modular and iterative PKS enzymes and the polyketides produced by those enzymes. Modular PKS enzymes are typically multi-protein complexes in which each protein contains multiple active sites, each of which is used only once during carbon chain assembly and modification. Iterative PKS enzymes are typically multi-protein complexes in which each protein contains only one or at most two active sites, each of which is used multiple times during carbon chain assembly and modification. As described in more detail below, a large number of the genes for both modular and aromatic PKS enzymes have been cloned.

Modular PKS genes are composed of coding sequences organized to encode a loading module, a number of extender modules, and a releasing domain. As described more fully below, each of these domains and modules corresponds to a polypeptide with one or more specific functions. Generally, the loading module is responsible for binding the first building block used to synthesize the polyketide and transferring it to the first extender module. The building blocks used to form complex polyketides are typically acylthioesters, most commonly acetyl, propionyl, malonyl, methylmalonyl, hydroxymalonyl, methoxymalonyl, and ethylmalonyl CoA. Other building blocks include amino acid and amino acid-like acylthioesters. PKSs catalyze the biosynthesis of polyketides through repeated, decarboxylative Claisen condensations between the acylthioester building blocks. Each module is responsible for binding a building block, performing one or more functions on that building block, and transferring the resulting compound to the next module. The next module, in turn, is responsible for attaching the next building block and transferring the growing compound to the next module until synthesis is complete. At that point, the releasing domain, often an enzymatic thioesterase (TE) activity, cleaves the polyketide from the PKS.

The polyketide known as 6-deoxyerythronolide B (6-dEB) is synthesized by a prototypical modular PKS enzyme. The genes, known as *eryAI, eryAII*, and *eryAIII*, that code for the multi-subunit protein known as deoxyerythronolide B synthase or DEBS (each subunit is known as DEBS1, DEBS2, or DEBS3) that synthesizes 6-dEB are described in U.S. Patent Nos. 5,672,491, 5,712,146 and 5,824,513.

The loading module of the DEBS PKS consists of an acyltransferase (AT) and an acyl carrier protein (ACP). The AT of the DEBS loading module recognizes propionyl CoA (other loading module ATs can recognize other acyl-CoAs, such as acetyl, malonyl, methylmalonyl, or butyryl CoA) and transfers it as a thioester to the ACP of the loading module. Concurrently, the AT on each of the six extender modules of DEBS recognizes a methylmalonyl CoA (other extender module ATs can recognize other CoAs, such as malonyl or alpha-substituted malonyl CoAs, i.e., malonyl, ethyLmalonyl, and 2-hydroxymalonyl CoA) and transfers it to the ACP of that module to form a thioester. Once DEBS is primed with propionyl- and methylmalonyl-ACPs, the acyl group of the loading module migrates to form a thioester (trans-esterification) at the KS of the first extender module; at this stage, module one possesses an acyl-KS adjacent to a methylmalonyl ACP. The acyl group derived from the DEBS loading module is then covalently attached to the alpha-carbon of the extender group to form a carbon-carbon bond, driven by concomitant decarboxylation, and generating a new acyl-ACP that has a backbone two carbons longer than the loading unit (elongation or extension). The growing polyketide chain is transferred from the ACP to the KS of the next module of DEBS, and the process continues.

The polyketide chain, growing by two carbons for each module of DEBS, is sequentially passed as a covalently bound thioester from module to module, in an assembly line-like process. The carbon chain produced by this process alone would possess a ketone at every other carbon atom, producing a polyketone, from which the name polyketide is derived. Commonly, however, additional enzymatic activities modify the beta keto group of the polyketide chain to which the two-carbon unit has been added before the chain is transferred to the next module. Thus, in addition to the minimal module containing KS, AT, and ACP necessary to form the carbon-carbon bond, modules may contain a ketoreductase (KR) that reduces the beta-keto group to an alcohol. Modules may also contain a KR plus a dehydratase (DH) that dehydrates the alcohol to a double bond. Modules may also contain a KR, a DH, and an enoylreductase (ER) that converts the double bond to a saturated single bond using the beta carbon as a methylene function. The DEBS modules include those with only a KR domain, only an inactive KR domain, and with all three KR, DH, and ER domains.

Once a polyketide chain traverses the final module of a PKS, it encounters the releasing domain, typically a thioesterase, found at the carboxyl end of most modular PKS enzymes. Here, the polyketide is cleaved from the enzyme and, for many but not all polyketides, cyclized. The polyketide can be further modified by tailoring or modification enzymes; these enzymes add carbohydrate groups or methyl groups, or make other modifications, i.e., oxidation or reduction, on the polyketide core molecule and/or substituents thereon. For example, 6-dEB is hydroxylated and glycosylated (glycosidated), and one of the glycosyl substituents methylated to yield the well known antibiotic erythromycin A in the *Saccharopolyspora erythraea* cells in which it is naturally produced.

While the above description applies generally to modular PKS enzymes and specifically to DEBS, there are a number of variations that exist in nature. For example, many PKS enzymes comprise loading modules that, unlike the loading module of DEBS, comprise an "inactive" KS domain that functions as a decarboxylase. This inactive KS is in most instances called KSQ, where the superscript is the single-letter abbreviation for the amino acid (glutamine) that is present instead of the active site cysteine required for ketosynthase activity. The epothilone PKS loading module contains a KS^{Y} domain in which tyrosine is present instead of the active site cysteine. Moreover, the synthesis of other polyketides begins with starter units that are unlike those bound by the DEBS or epothilone loading modules. The enzymes that bind such starter units can include, for example, an AMP ligase such as that employed in the biosynthesis of FK520, FK506, and rapamycin, a non-ribosomal peptide synthase (NRPS) such as that employed in the biosynthesis of leinamycin, or a soluble CoA ligase.

Other important variations in PKS enzymes relate to the types of building blocks incorporated as extender units. As for starter units, some PKS enzymes incorporate amino acid like acylthioester building blocks using one or more NRPS modules as extender modules. The epothilone PKS, for example, contains an NRPS module. Another such variation is found in the FK506, FK520, and rapamycin PKS enzymes, which contain an NRPS that incorporates a pipecolate residue and also serves as the releasing domain of the PKS. Yet another variation relates to additional activities in an extender module. For example; one module of the epothilone PKS contains a methyltransferase (MT) domain, which incorporates a methyl group into the polyketide.

Recombinant methods for manipulating modular and iterative PKS genes are described in U.S. Patent Nos. 5,962,290; 5,672,491; 5,712,146; 5,830,750; and 5,843,718; and in PCT patent publication Nos. 98/49315 and 97/02358. These and other patents describe recombinant expression vectors for the heterologous production of polyketides as well as recombinant PKS genes assembled by combining parts of two or more different PKS genes or gene clusters that produce novel polyketides. To date, such methods have been used to produce known or novel polyketides in organisms such as *Streptomyces,* which naturally produce polyketides, and *E. coli* and yeast, which do not naturally produce polyketides (see U.S. Patent No. 6,033,883, incorporated herein by reference). In the latter hosts, polyketide production is dependent on the heterologous expression of a phosphopantetheinyl transferase, which activates the ACP domains of the PKS (see PCT publication No. 97/13845.

While such methods are valuable and highly useful, certain polyketides are expressed only at very low levels in, or are toxic to, the heterologous host cell employed. As an example, the anticancer agents epothilones A, B, C, and D were produced in *Streptomyces* by heterologous expression of the epothilone PKS genes (Tang *et al.,* 28 Jan. 00, Cloning and heterologous expression of the epothilone gene cluster, *Science, 287*:640-642, and PCT Pub. No. 00/031247). Epothilones A and B were produced at less than about 50 to 100 µg/L and appeared to have a deleterious effect on the producer cells.

Epothilones A and B were first identified as an antifungal activity extracted from the myxobacterium *Sorarcgium cellulosum* (see Gerth *et al*., 1996, *J. Antibiotics* 49: 560-563 and Germany Patent No. DE 41 38 042) and later found to have activity in a tubulin polymerization assay (see Bollag *et al*., 1995, *Cancer Res. 55*:2325-2333). Epothilones A and B and certain naturally occurring and synthetic derivatives have since been extensively studied as potential antitumor agents for the treatment of cancer. The chemical structures of the epothilones produced by *Sorangium cellulosum* strain So ce 90 were described in Hofle *et al*., 1996, Epothilone A and B - novel 16-membered macrolides with cytotoxic activity: isolation, crystal structure, and conformation in solution, *Angew. Chem. Int. Ed. Engl.* 35(13/14): 1567-1569. Epothilones A (R = H) and B (R = CH₃) have the structure shown below and show broad cytotoxic activity against eukaryotic cells and noticeable activity and selectivity against breast and colon tumor cell lines.

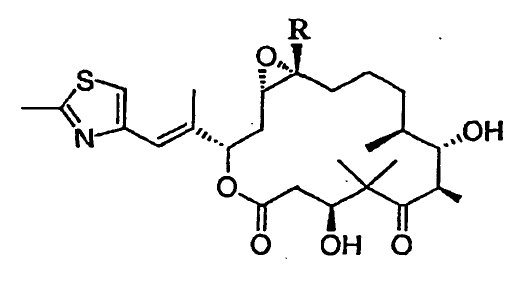

The desoxy counterparts of epothilones A and B, also known as epothilones C (R = H) and D (R = CH₃), have been chemically synthesized *de novo* but are also present as minor products in fermentations of *S. cellulosum*. Epothilones C and D are less cytotoxic than epothilones A and B; their structures are shown below.

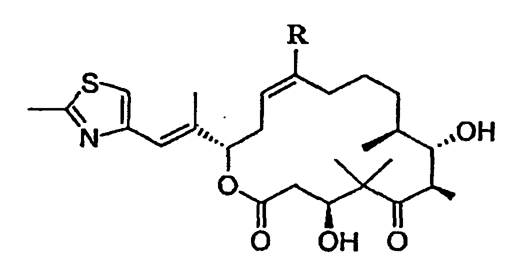

Other naturally occurring epothilones have been described. These include epothilones E and F, in which the methyl side chain of the thiazole moiety of epothilones A and B has been hydroxylated to yield epothilones E and F, respectively, as well as many other epothilone compounds (see PCT Pub. No. 99/65913).

Because of the potential for use of the epothilones as anticancer agents, and because of the low levels of epothilone produced by the native So ce 90 strain, a number of research teams undertook the effort to synthesize the epothilones. As noted above, this effort has been successful (see Balog et *al*., 1996, Total synthesis of (-)-epothilone A, *Angew. Chem. Int.* Ed. *Engl.* 35(23/24): 2801-2803; Su *et al*., 1997, Total synthesis of (-)-epothilone B: an extension of the Suzuki coupling method and insights into structure-activity relationships of the epothilones, *Angew. Chem.* Int. Ed. Engl. 36(7): 757-759; Meng *et al*., 1997, Total syntheses of epothilones A and B, *JACS* 119(42): 10073-10092; and Balog *et al*., 1998, A novel aldol condensation with 2-methyl-4-pentenal and its application to an improved total synthesis of epothilone B, *Angew. Chem. Int*. *Ed.* Engl. 37(19): 2675-2678). Despite the success of these efforts, the chemical synthesis of the epothilones is tedious, time-consuming, and expensive. Indeed, the methods have been characterized as impractical for the full-scale pharmaceutical development of any epothilone as an anticancer agent.

A number of epothilone derivatives, as well as epothilones A - D, have been studied *in vitro* and in *vivo* (see Su et *al*., 1997, Structure-activity relationships of the epothilones and the first in *vivo* comparison with paclitaxel, *Angew. Chem. Int. Ed. Engl.* 36(19): 2093-2096; and Chou et *al*., Aug. 1998, Desoxyepothilone B: an efficacious microtubule-targeted antitumor agent with a promising *in vivo* profile relative to epothilone B, Proc. *Natl. Acad. Sci. USA 95:* 9642-9647). Additional epothilone derivatives and methods for synthesizing epothilones and epothilone derivatives are described in PCT Pub. Nos. 00/23452, 00/00485, 99/67253, 99/67252, 99/65913, 99/54330, 99/54319, 99/54318, 99/43653, 99/43320, 99/42602, 99/40047, 99/27890, 99/07692, 99/02514, 99/01124,98/25929, 98/22461, 98/08849, and 97/19086; U.S. Patent No. 5,969,145; and Germany patent publication No. DE 41 38 042, WO 00/22139 discloses a cloned Sorangium cellulosum polyletide synthase (PKS) biosynthetic cluster DNA.

There remains a need for economical means to produce not only the naturally occurring epothilones but also the derivatives or precursors thereof, as well as new epothilone derivatives with improved properties. There remains a need for a host cell that produces epothilones or epothilone derivatives that is easier to manipulate and ferment than the natural producer *Sorangium cellulosum* and that produces more of the desired polyketide product. The present invention meets these needs by providing host cells that produce polyketides at high levels and are useful in the production of not only epothilones, including new epothilone derivatives described herein, but also other polyketides.

### Summary of the Invention

The present invention provides recombinant host cells of the suborder *Cystobacterineae* containing recombinant expression vectors that encode heterologous PKS genes and produce polyketides synthesized by the PKS enzymes encoded by the genes on those vectors.

In one aspect of the present invention, there is provided a recombinant host cell of the suborder *Cystobacterineae* containing a recombinant expression vector that encodes an epothilone polyketide synthase (PKS) gene and produces a polyketide synthesized by a PKS enzyme encoded on said vector, wherein said cell is *Myxococcus xanthus* K111-40.1 (ATCC accession no. PTA-2712), *Myxococcus xanthus* K111-72.4.4 (ATCC accession No. PTA-2713) or *Myxococcus xanthus* strain K90-132.1.1.2 (ATCC accession No. PTA-2715).

We also describe a recombinant host cell of the suborder *Cystobacterineae* containing a recombinant expression vector that encodes a heterologous epothilone polyketide synthase (PKS) gene and produces a polyketide synthesized by a PKS enzyme encoded on said vector and further comprises a heterologous gene that encodes MtaA, MatB or MatC.

We also describe recombinant DNA vectors capable of chromosomal integration or extrachromosomal replication in the host cells of the invention. These vectors may comprise at least a portion or a PKS coding sequence and are capable of directing expression of a functional PKS enzyme in the host cells of the invention. In a related embodiment, we also describe vectors and host cells that comprise the genes and gene products required to produce a substrate for polyketide biosynthesis that is either not produced or is produced in low abundance in a host cell of the invention. In one embodiment, the genes and gene products catalyse the synthesis of ethylmalonyl CoA. In another embodiment, the genes and gene products catalyse the synthesis of butyryl CoA.

We also describe a method for producing a polyketide in a host cell of the suborder *Cystobacterinene*, which polyketide is not naturally produced in said host cell, said method comprising culturing the host cell transformed with a recombinant DNA vector of the invention under conditions such that a PKS gene encoded on the vector is expressed and said polyketide is produced. The host cells of the invention may be fermented to produce polyketides in high yied.

In a preferred embodiment, the recombinant host cell of the invention produces epothilone or an epothilone derivative. Thus, the present invention provides recombinant host cells that produce a desired epothilone or epothilone derivative. In a preferred embodiment, the host cell produces one or more epothilones at equal to or greater than 10 mg/L. In one embodiment, the invention provides host cells that produce one or more epothilones at levels higher than the levels produced in a naturally occurring organism that produces epothilones. In another embodiment, the invention provides host cells that produce mixtures of epothilones that are less complex than the mixtures produced by a naturally occurring host cell that produces epothilones. The recombinant host cells of the invention also include host cells that produce only one desired epothilone or epothilone derivative as a major product.

We also describe recombinant DNA expression vectors that encode all or a portion of the epothilone PKS. Thus, the present invention provides recombinant DNA expression vectors that encode the proteins required to produce epothilones A, B, C, and D in the host cells of the invention. The present invention also provides recombinant DNA expression vectors that encode portions of these proteins. Recombinant DNA compounds are also described that encode a hybrid protein, which hybrid protein includes all or a portion of a protein involved in epothilone biosynthesis and all or a portion of a protein involved in the biosynthesis of another polyketide or non-ribosomal-derived peptide.

### Brief Description of the Figures

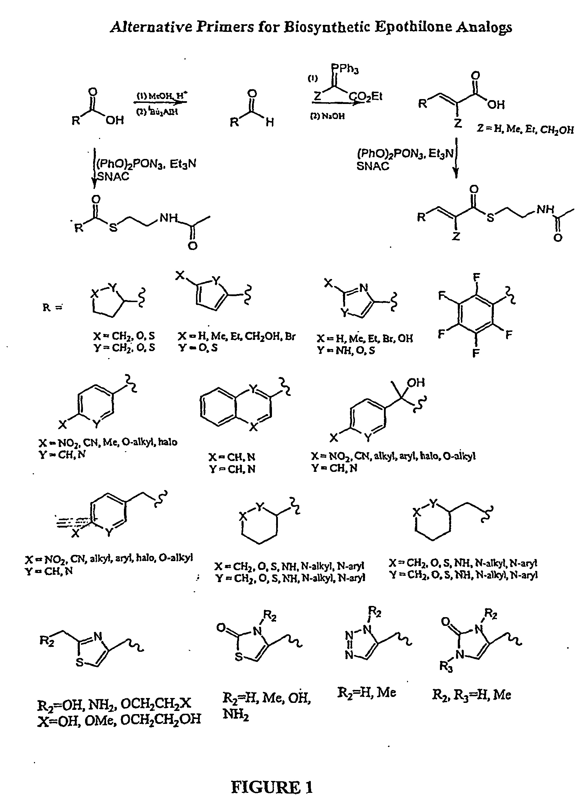

Figure 1 shows a number of precursor compounds to N-acetyl cysteamine thioester derivatives that can be supplied to an epothilone PKS of the invention in which the NRPS-like module one or module two KS domain has been inactivated to produce a novel epothilone derivative. A general synthetic procedure for making such compounds is also shown.

Figure 2 shows restriction site and function maps of plasmids pKOS35-82.1 and pKOS35-82.2.

Figure 3 shows restriction site and function maps of plasmids pKOS35-154 and pKOS90-22.

Figure 4 shows a schematic of a protocol for introducing the epothilone PKS and modification enzyme genes into the chromosome of a *Myxococcus xanthus* host cell as described in Example 2.

Figure 5 shows a map of pBeloBACII as described in Example 2.

Figure 6 shows the baseline performance of *Myxococcrcs xanthus* strain K111-40-1 in a simple production medium consisting only of 5 g/L casitone (a pancreatic casein digest) and 2 g/L magnesium sulfate. Legend: Growth (•), production (■), and ammonia generation (▲) profiles for the basal CTS medium in a batch process; culture conditions were as described in Materials and Methods in Example 3.

Figure 7 shows the effect of XAD-16 resin on the fermentation performance of *Myxococcus xanthus* strain K111-40-1 in CTS production medium. **Legend:** Growth (•) and production (■) profiles with the incorporation of 20 g/L XAD-16 resin to the CrS production medium in a batch process.

Figure 8 shows the influence of casitone on growth and product yield. **Legend:** Effect of casitone concentration on growth (•), production (■), and specific productivity (▲).

Figure 9 shows the influence of trace elements and higher methyl oleate concentrations on growth and product yield. **Legend:** Effect of methyl oleate (•) and trace elements (■) on production.

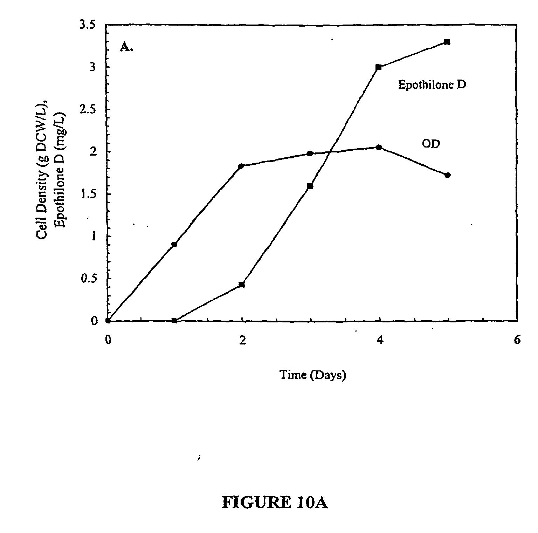

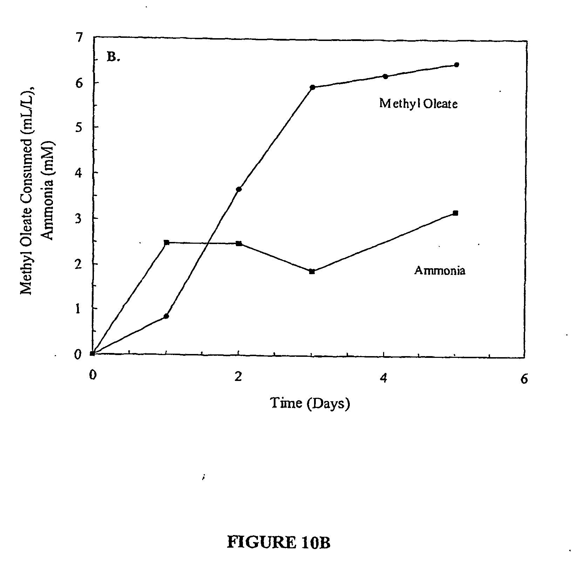

Figure 10A shows the growth and production of the *M*. *x*antitus strain in the presence of optimal concentrations of methyl oleate and trace elements in a batch fermentation process. Exponential growth of the occurred during the first two days after inoculation. Production of epothilone D began at the onset of the stationary phase and ceased when cell lysis occurred with the depletion of methyl oleate on day 5. Figure 10B shows the time courses corresponding to the consumption of methyl oleate and generation of ammoniaduring the growth and proudction of the *M. xanthus* strain in the presence of optimal concentrations of methyl oleate and trace elements in a batch fermentation process. Legend: A.) Growth (•) and production (■) profiles with the addition of optimal concentrations of methyl oleate (7 mL/L) and trace elements (4 mL/L) to the CTS production medium in a batch process. B.) Time courses corresponding to the consumption of methyl oleate (•) and the generation of ammonia (■).

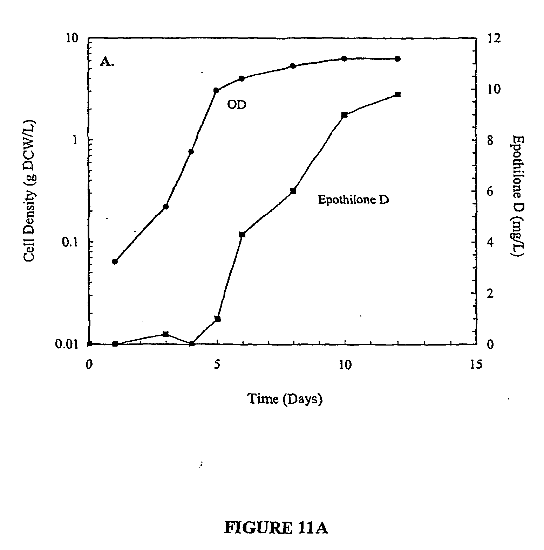

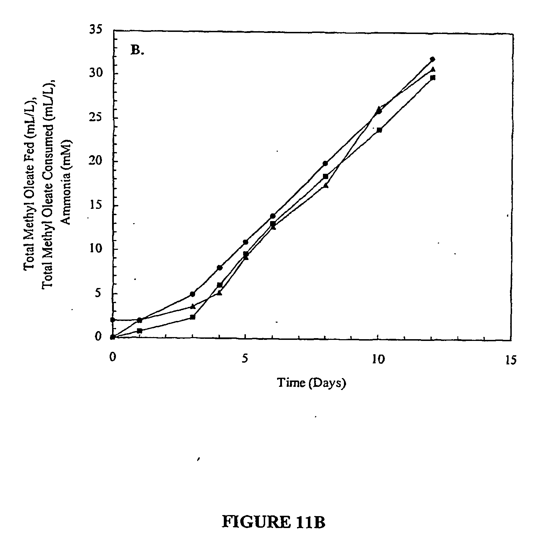

Figure 11A shows the influence of intermittent fed-batch process on the growth and production of the M. *xanthus* strain. **Legend:** Growth (•) and production (■) profiles for the intermittent fed-batch process in shake-flasks. The casitone and methyl oleate feed rates were 2 g/L/day and 3 mL/L/day, respectively. Figure 11B shows the constant rates of consumption of methyl oleate and generation of ammonia during the course of the fermentation. Legend: Time courses corresponding to the total addition of methyl oleate to the cultures (•), the total consumption of methyl oleate (■), and the generation of ammonia (▲).

Figure 12 shows the production profile for the intermittent fed-batch process in a 5-L bioreactor. The casitone and methyl oleate feed rates were 2 g/L/day and 3 mL/L/day, respectively.

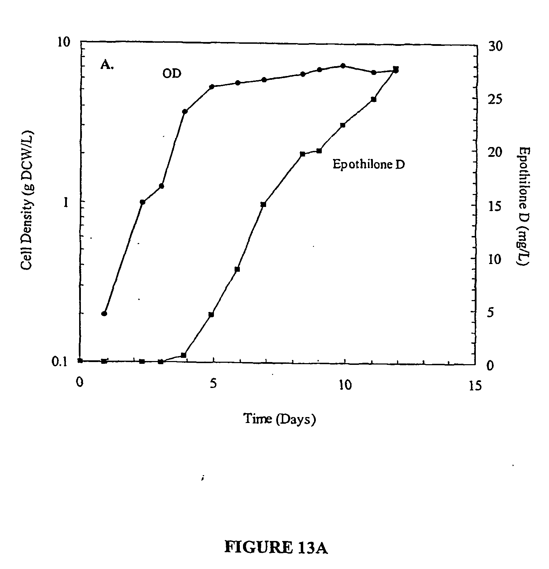

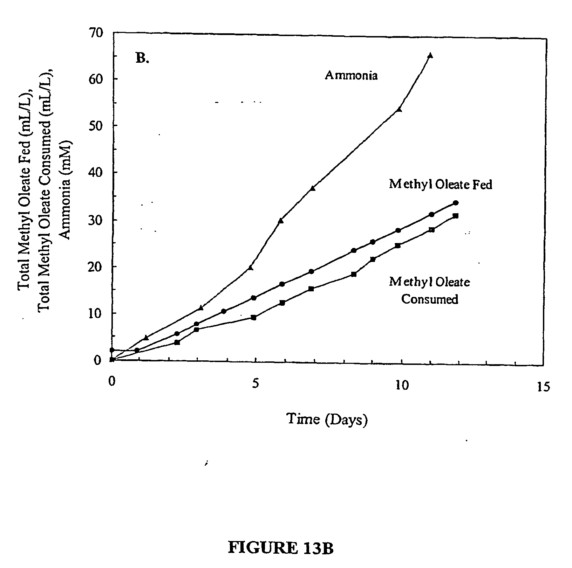

Figure 13A shows the impact of continous feeds on growth and production. Legend: Growth (•) and production (■) profiles for the continuous fed-batch process in a 5-L bioreactor. The casitone and methyl oleate feed rates were 2 g/L/day and 3 mL/L/day, respectively. Figure 13B shows the time course of methyl oleate addition and consumption as well as the generation of ammonia during the continuous fed-batch process. **Legend:** Time courses corresponding to the total addition of methyl oleate to the cultures (•), the total consumption of methyl oleate (■), and the generation of ammonia (▲); culture conditions were as described in Materials and Methods

### Detailed Description of the Invention

Statements regarding the scope of the present invention and definitions of terms used herein are listed below. The definitions apply to the terms as they are used throughout this specification, unless otherwise limited in specific instances, either individually or as part of a larger group.

The term "isolated" as used herein to refer to a compound of the present invention, means altered "by human intervention from its natural state. For example, if the compound occurs in nature, it has been changed or removed from its original environment, or both. In other words, a compound naturally present in a living organism is not "isolated," but the same compound separated from the coexisting materials of its natural state is "isolated", as the term is employed herein. The term "isolated" can also mean a compound that is in a preparation that is substantially free of contaminating or undesired materials. With respect to compounds found in nature, substantially free of the materials with which that compound or composition is associated in its natural state.

The term "purified" as it refers to a compound means that the compound is in a preparation in which the compound forms a major component of the preparation, such as constituting about 50%, about 60%, about 70%, about 80%, about 90%, about 95% or more by weight of the components in the preparation.

Described herein are recombinant methods and materials for producing polyketides in recombinant host cells; recombinant host cells that produce polyketides; novel polyketides related in structure to the epothilones; methods for purifying epothilones; and crystalline forms of epothilone D.

In one embodiment, the present invention provides recombinant host cells of the suborder *Cystobacterineae* containing recombinant expression vectors that encode heterologous PKS genes and produce polyketides synthesized by the PKS enzymes encoded on those vectors. As used herein, the term recombinant refers to a cell, compound, or composition produced by human intervention, typically by specific and directed manipulation of a gene or portion thereof. The suborder *Cystobacterineae* is one of two (the other is *Sorangineae,* which includes the epothilone producer *Sorangium cellulosum*) in the order *Myxococcales*. The suborder. *Cystobacterineae* includes the family *Myxococcacecre* and the family *Cystobacteraceae.* The family *Myxococcacceae* includes the genus *Angiococcus* (i.e., *A. disciformis*), the genus *Myxococcus,* and the genus *Corallococcus* (i.e., *C. macrosporus, C. corralloides*, and C. *exiguus*). The family *Cystobacteraceae* includes the genus *Cystobacter* (i.e., *C*. *fuscus, C. ferrugineus, C. minor, C. velatus*, and *C*. *violaceus*), the genus *Melittangium* (i.e., *M. boletus* and M. *lichenicola*), the genus *Stigmatella* (i.e., *S*. *erecta* and *S. aurantiaca*), and the genus *Archangium* (i.e., *A. gephyra*). Especially preferred host cells of the invention are those that produce a polyketide at equal to or greater than 10 to 20 mg/L, more preferably at equal to or greater than 100 to 200 mg/L, and most preferably at equal to or greater than 1 to 2 g/L.

Described herein are host cells from the genus *Myxococcus* or the genus *Stigmatella*, such as host cells selected from the group consisting of M. *stipitatus,* M. *fulvus, M*. *xanthus, M. virescens, S. erecta*, and S. *aurantiaca*. Especially preferred *Myxococcus* host cells of the invention are those that produce a polyketide at equal to or greater than 10 to 20 mg/L, more preferably at equal to or greater than 100 to 200 mg/L, and most preferably at equal to or greater than 1 to 2 g/L. Especially preferred are M. *xanthus* host cells that produce at these levels. M. *xanthus* host cells described herein include the DZ1 cell line (Campos *et al*., 1978, J. *Mol. Biol. 119*: 167-178), the TA-producing cell line ATCC 31046, the DK1219 cell line (Hodgkin and Kaiser, 1979, *Mol. Gen. Genet*. *171*: 177-191), the DK1622 cell line (Kaiser, 1979, Proc. *Natl.* Acad. Sci. USA 76: 5952-5956. Host cells of the invention are M-xanthus cells of strains k111-40.1, k111-72.4.4 and k90-132.1.1.2.

The host cells of the invention comprise a recombinant DNA expression vector. Recombinant DNA vectors may be capable of chromosomal integration or extrachromosomal replication in these host cells. Such vectors may comprise at least a portion of a PKS coding sequence and be capable of directing expression of a functional PKS enzyme in the host cells of the invention. As used herein, the term expression vector refers to any nucleic acid that can be introduced into a host cell. An expression vector can be.maintained stably or transiently in a cell, whether as part of the chromosomal or other DNA in the cell or in any cellular compartment, such as a replicating vector in the cytoplasm. An expression vector also comprises a gene that serves to direct the synthesis of RNA that is translated into a polypeptide in the cell or cell extract. Thus, the vector either includes a promoter to enhance gene expression or is integrated into a site in the chromosome such that gene expression is obtained. Furthermore, expression vectors typically contain additional functional elements, such as resistance-conferring genes to act as selectable markers and regulatory genes to enhance promoter activity.

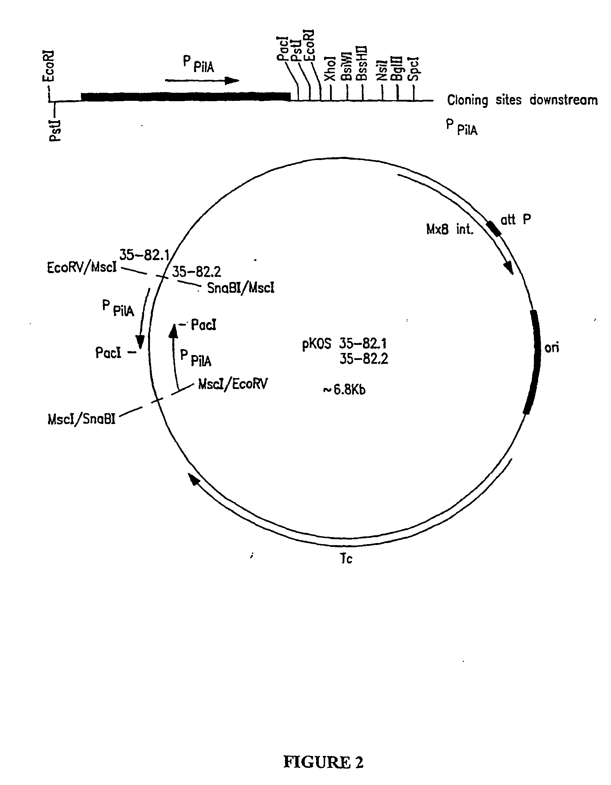

Expression vectors may be used in preparing recombinant *Myxococcus xanthus* expression vectors and the host cells of the invention. These vectors, designated plasmids pKOS35-82.1 and pKOS35-82.2 (Figure 2), are able to replicate in E. *coli* host cells as well as integrate into the chromosomal DNA of M. *xanthus*. The vectors comprise the Mx8 attachment and integration genes as well as the *pilA* promoter with restriction enzyme recognition sites placed conveniently downstream. The two vectors differ from one another merely in the orientation of the *pilA* promoter on the vector and can be readily modified to include epothilone PKS and modification enzyme genes or other PKS and modification enzyme genes. The construction of the vectors is described in Example 1.

The host cells of the invention can be used not only to produce a polyketide found in nature but also to produce polyketides produced by the products of recombinant PKS genes and modification enzymes. Recombinant DNA expression vectors may be used that comprise a hybrid PKS. For purposes of the present invention a hybrid PKS is a recombinant PKS that comprises all or part of one or more extender modules, loading module, and thioesterase/cyclase domain of a first PKS and all or part of one or more extender modules, loading module, and thioesterase/cyclase domain of a second PKS.

Those of skill in the art will recognize that all or part of either the first or second PKS in a hybrid PKS need not be isolated from a naturally occurring source. For example, only a small portion of an AT domain determines its specificity. See PCT Pub. No. 00/001838. The state of the art in DNA synthesis allows the artisan to construct *de novo* DNA compounds of size sufficient to construct a useful portion of a PKS module or domain. For purposes of the present invention, such synthetic DNA compounds are deemed to be a portion of a PKS.

There are a wide variety of PKS genes that serve as readily available sources of DNA and sequence information for use in constructing hybrid PKS-encoding DNA compounds. Methods for constructing hybrid PKS-encoding DNA compounds are described in U.S. Patent Nos. 6,022,731; 5,962,290; 5,672,491; and 5,712,146 and PCT Pub. Nos. 98/49315; 99/61599; and 00/047724. The hybrid PKS-encoding DNA compounds can be hybrids of more than two PKS genes. Even where only two genes are used, there are often two or more modules in the hybrid gene in which all or part of the module is derived from a second PKS gene. Those of skill in the art will appreciate that a hybrid PKS includes, but is not limited t,o a PKS of any of the following types: (i) a PKS that contains a module in which at least one of the domains is from a heterologous module; (ii) a PKS that contains a module from a heterologous PKS; (iii) a PKS that contains a protein from a heterologous PKS; and (iv) combinations of the foregoing.

Hybrid PKS enzymes are often constructed by replacing coding sequences for one or more domains of a module from a first PKS with coding sequences for one or more domains of a module from a second PKS to construct a recombinant coding sequence. Generally, any reference herein to inserting or replacing a KR, DH, and/or ER domain includes the replacement of the associated KR, DH, or ER domains in that module, typically with corresponding domains from the module from which the inserted or replacing domain is obtained. The KS and/ or ACP of any module can also be replaced, if desired or beneficial, with another KS and/or ACP. For example, if the production of an epothilone derivative compound is low due to an alteration in a module, production may be improved by altering the KS and/ or ACP domains of the succeeding module. In each of these replacements or insertions, the heterologous KS, AT, DH, KR, ER, or ACP coding sequence can originate from a coding sequence from another module of the same or different PKS or from chemical synthesis to obtain the hybrid PKS coding sequence.

While hybrid PKS genes may be used, recombinant PKS genes may also be used in which there is no second PKS gene sequence present but which differ from a naturally occurring PKS gene by one or more mutations and/or deletions. The deletions can encompass one or more modules or domains and/or can be limited to a deletion within one or more modules or domains. When a deletion encompasses an entire extender module (other than an NRPS module), the resulting polyketide derivative is at least two carbons shorter than the compound produced from the PKS from which the deleted version was derived. The deletion can also encompass an NRPS module and/or a loading module. When a deletion is within a module, the deletion may encompass only a single domain, typically a KR, DH, or ER domain, or more than one domain, such as both DH and ER domains, or both KR and DH domains, or all three KR, DH, and ER domains. A domain of a PKS can also be "deleted" functionally by mutation, such as by random or site-specific mutagenesis. Thus, as exemplified herein, a KR domain can be rendered non-functional or less than fully functional by mutation. Moreover, the specificity of an AT domain can also be altered by mutation, such as by random or site-specific mutagenesis.

In a preferred and illustrative embodiment, the recombinant host cell of the invention produces epothilone or an epothilone derivative. The naturally occurring epothilones (including epothilone A, B, C, D, E, and F) and non naturally occurring compounds structurally related thereto (epothilone derivatives or analogs) are potent cytotoxic agents specific for eukaryotic cells. These compounds have application as anti-fungals, cancer chemotherapeutics, and immunosuppressants, and generally for the treatment of inflammation or any hyperproliferative disease, such as psoriasis, multiple sclerosis, atherosclerosis, and blockage of stents. The epothilones are produced at very low levels in the naturally occurring *Sorangitim cellulosum* cells in which they have been identified. Moreover, S. *cellulosum* is very slow growing, and fermentation of *S. cellulosum* strains is difficult and time-consuming. One important benefit conferred by the present invention is the ability simply to produce an epothilone or epothilone derivative in a non-*S. cellulosum* host cell. Another advantage of the present invention is the ability to produce the epothilones at higher levels and in greater amounts in the recombinant host cells provided by the invention than possible in the naturally occurring epothilone producer cells. Yet another advantage is the ability to produce an epothilone derivative in a recombinant host cell. Thus, the present invention provides recombinant host cells that produce a desired epothilone or epothilone derivative. In a preferred embodiment, the host cell produces the epothilone or epothilone derivative at equal to or greater than 10 mg/L. In one embodiment, the invention provides host cells that produce one or more of the epothilones or epothilone derivatives at higher levels than produced in the naturally occurring organisms that produce epothilones. The host cells may produce mixtures of epothilones that are less complex than the mixtures produced by naturally occurring host cells that produce epothilones.

The epothilone PKS and modification enzyme genes were cloned from the epothilone producing strain, *Sorangium cellulosum* SMP44. Total DNA was prepared from this strain using the procedure described by Jaoua *et al*., 1992, *Plasmid 28*: 157-165. A cosmid library was prepared from *S. cellulosum* genomic DNA in pSupercos (Stratagene). The entire PKS and modification enzyme gene cluster was isolated in four overlapping cosmid clones (deposited on February 17,1999, under the terms of the Budapest Treaty with the American Type Culture Collection (ATCC), 10801 University Blvd., Manassas, VA, 20110-2209 USA, and assigned ATCC accession numbers as follows: pKOS35-70.1A2 (ATCC 203782), pKOS35-70.4 (ATCC 203781), pKOS35-70.8A3 (ATCC 203783), and pKOS35-79.85 (ATCC 203780)) and the DNA sequence determined, as described in PCT Pub. No. 00/031237. DNA sequence analysis revealed a PKS gene cluster with a loading module and nine extender modules. Downstream of the PKS sequence is an open reading frame (ORF), designated *epoK*, that shows strong homology to cytochrome P450 oxidase genes and encodes the epothilone epoxidase modification enzyme.

The PKS genes are organized in 6 ORFs. At the polypeptide level, the loading module and extender modules 1 (an NRPS), 2, and 9 appear on individual polypeptides; their corresponding genes are designated *epoA, epoB, epoC,* and *epoF* respectively. Modules 3, 4, 5, and 6 are contained on a single polypetide whose gene is designated *epoD*, and modules 7 and 8 are on another polypeptide whose gene is designated *epoE*. The spacing between ORFs suggests that *epoC, epoD, epoE* and *epoF* constitute an operon. The *epoA, epoB*, and *epoK* gene may be also part of this large operon, but there are spaces of approximately 100 bp between *epoB* and *epoC* and 115 bp between *epoF* and *epoK* that could contain a promoter. The epothilone PKS gene cluster is shown schematically below in Scheme 1.

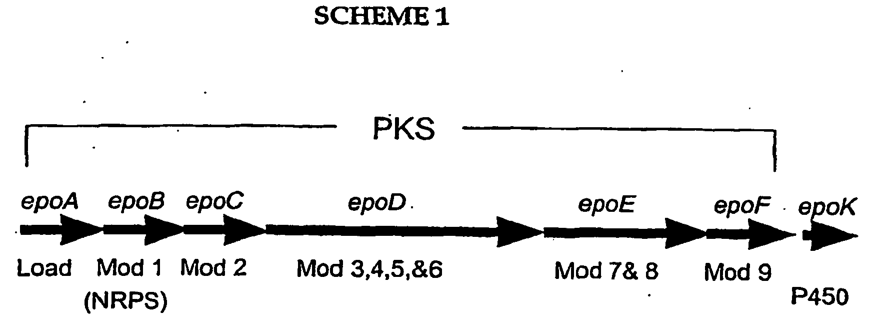

Immediately downstream of *epoK*, the P450 epoxidase gene, is ORF1, which encodes a polypeptide that appears to include membrane spanning domains and may be involved in epothilone transport. This ORF is followed by a number of ORFs that include genes that may encode proteins involved in transport and regulation.
A detailed examination of the modules shows an organization and composition consistent with the biosynthesis of epothilone. The description that follows is at the polypetide level. The sequence of the AT domain in the loading module and in extender modules 3, 4, 5, and 9 shows similarity to the consensus sequence for malonyl loading modules, consistent with the presence of an H side chain at C-14, C-12 (epothilones A and C), C-10, and C-2, respectively, as well as the loading module. The AT domains in modules 2, 6, 7, and 8 resemble the consensus sequence for methylmalonyl specifying AT domains, again consistent with the presence of methyl side chains at C-16, C-8, C-6, and C-4 respectively.

The loading module contains a KS domain in which the cysteine residue usually present at the active site is instead a tyrosine. This domain is designated as KS^{y} and serves as a decarboxylase, which is part of its normal function, but cannot function as a condensing enzyme. Thus, the loading module is expected to load malonyl CoA, move it to the ACP, and decarboxylate it to yield the acetyl residue required for condensation with cysteine. Extender module 1 is the non-ribosomal peptide synthetase that activates cysteine and catalyzes the condensation with acetate on the loading module. The sequence contains segments highly similar to ATP-binding and ATPase domains, required for activation of amino acids, a phosphopantetheinylation site, an oxidation domain, a cyclization domain, and an elongation domain. Extender module 2 determines the structure of epothilone at C-15-C-17. The presence of the DH domain in module 2 yields the C-16-17 dehydro moiety in the molecule. The domains in module 3 are consistent with the structure of epothilone at C-14 and C-15; the OH that comes from the action of the KR is employed in the lactonization of the molecule. Extender module 4 controls the structure at C-12 and C-13 where a double bond is found in epothilones C and D. Although the sequence of the AT domain appears to resemble those that specify malonate loading, it can also load methylmalonate, thereby accounting in part for the mixture of epothilones found in the fermentation broths of the naturally producing organisms.

A significant departure from the expected array of functions was found in extender module 4. This module was expected to contain a DH domain, thereby directing the synthesis of epothilones C and D as the products of the PKS. Analysis revealed that the space between the AT and KR domains of module 4 was not large enough to accommodate a functional DH domain. Thus, the extent of reduction at module 4 appears not to proceed beyond the ketoreduction of the beta-keto formed after the condensation directed by extender module 4. As shown herein, the epothilone PKS genes alone are sufficient to confer the ability to produce epothilones C and D to the host cells of the invention. The heterologous production of epothilones C and D demonstrates that there must be a dehydratase function that introduces the double bond. Based on heterologous expression of the epothilone PKS genes and the products produced by altered epothilone PKS genes, the dehydration reaction that forms this double bond is believed to be mediated by the DH domain of extender module 5 of the epothilone PKS and the generation of a conjugated diene precursor prior to reduction by the ER domain of module 5.

Extender modules 5 and 6 each have the full set of reduction domains (KR, DH and ER) to yield the methylene functions at C-11 and C-9. Extender modules 7 and 9 have KR domains to yield the hydroxyls at C-7 and C-3, and extender module 8 does not have a functional KR domain, consistent with the presence of the keto group at C-5. Extender module 8 also contains a methyltransferase (MT) domain that results in the presence of the geminal dimethyl function at C-4. Extender module 9 also has a thioesterase domain that terminates polyketide synthesis and catalyzes ring closure.

The genes, proteins, modules, and domains of the epothilone PKS are summarized in the following Table 2.

**TABLE 2**

| **Gene** | **Protein** | **Modules** | **Domains Present** |
|---|---|---|---|
| *epoA* | EpoA . | Load | KS^{Y} mAT ER ACP |
| *epoB* | EpoB | 1 | NRPS, condensation, heterocyclization, |
| | | | adenylation, thiolation, PCP |
| *epoC* | EpoC | 2 | KS mmAT DH KR ACP |
| *epoD* | EpoD | 3-6 | KS mAT KR ACP; KS mAT KR ACP; KS |
| | | | mAT DH ER KR ACP; KS mmAT DH ER |
| | | | KR ACP |
| *epoE* | EpoE | 7-8 | KS mmAT KR ACP; KS mmAT MT DH* KR* AC |
| *epoF* | EpoF | 9 | KS mAT KR DH* ER* ACP TE |
| NRPS - non-ribosomal peptide synthetase; KS - ketosynthase; mAT - malonyl CoA specifying acyltransferase; mmAT - methylmalonyl CoA specifying acyltransferase; DH - dehydratase; enoylreductase; KR - ketoreductase; MT - methyltransferase; TE thioesterase; | | | |

| | | | |
|---|---|---|---|
| * - inactive domain. | | | |

Inspection of the sequence has revealed translational coupling between *epoA* and *epoB* (loading module and the extender module 1 NRPS) and between *epoC* and *epoD*. Very small gaps are seen between *epoD* and *epoE* and *epoE* and *epoF* but gaps exceeding 100 bp are found between *epoB* and *epoC* and *epoF* and *epoK*. These intergenic regions may contain promoters.

Thus, the epothilone PKS is a multiprotein complex composed of the gene products of the *epoA, epoB, epoC, epoD, epoE,* and *epoF* genes. To confer the ability to produce epothilones to a host cell, one provides the host cell with the recombinant *epoA, epoB, epoC, epoD, epoE,* and *epoF* genes of the present invention, and optionally other genes, such as *epoK*, capable of expression in that host cell. Those of skill in the art will appreciate that, while the epothilone and other PKS enzymes may be referred to as a single entity herein, these enzymes are typically multisubunit proteins. Thus, one can make a derivative PKS (a PKS that differs from a naturally occurring PKS by deletion or mutation) or hybrid PKS (a PKS that is composed of portions of two different PKS enzymes) by altering one or more genes that encode one or more of the multiple proteins that constitute the PKS.

The post-PKS modification or tailoring of epothilone includes multiple steps mediated by multiple enzymes. These enzymes are referred to herein as tailoring or modification enzymes. Expression of the epothilone PKS genes *epoA, epoB, epoC, epoD, epoE,* and *epoF* in host cells of the invention that do not express *epoK* leads to the production of epothilones C and D as major products, which lack the C-12-C-13 epoxide of epothilones A and B, having instead a C-12-C-13 double bond. Thus, epothilones C and D are converted to epothilones A and B by an epoxidase encoded by the *epoK* gene. Epothilones A and B may be converted to epothilones E and F by a hydroxylase gene, which may be encoded by a gene associated with the epothilone PKS gene cluster or by another gene endogenous to *Sorangium cellulosum*. Alternatively, these compounds may be formed by the loading module binding a starter unit other than malonyl CoA (such as hydroxymalonyl CoA). Thus, one can produce an epothilone or epothilone derivative modified as desired in a host cell by providing that host cell with one or more recombinant modification enzyme genes or by utilizing a host cell that naturally expresses (or does not express) the modification enzyme and/ or by providing starter units other than malonyl CoA.

Thus, a wide variety of recombinant DNA compounds and host cells may be provided for expressing the naturally occurring epothilones A, B, C, and D and derivatives thereof. Recombinant host cells may also produce epothilone derivatives modified in a manner similar to epothilones E and F. Moreover, any epothilone or epothilone derivative described herein can be converted to the corresponding epothilone E or F derivative in accordance with the methods described in PCT Pat. Pub. No. 00/ 039276.

A wide variety of recombinant DNA compounds and host cells that make epothilone derivatives may also be provided. As used herein, the phrase epothilone derivative refers to a compound that is produced by a recombinant epothilone PKS in which at least one domain has been inserted or in which a domain has either been rendered inactive by deletion or mutation, mutated to alter its catalytic function, or replaced by a domain with a different function. In any event; the epothilone derivative PKS so produced functions to produce a compound that differs in structure from a naturally occurring epothilone selected from the group consisting of epothilones A, B, C, D, E, and F.

The host cells of the invention can be grown and fermented under conditions known in the art for other purposes to produce compounds.

Also described herein are novel methods for fermenting the host cells of the invention. The compounds can be isolated from the fermentation broths of these cultured cells and purified by methods such as those in Example 3, below.

Described herein are a number of methods relating to the fermentation of *Myxococcus* strains for production of polyketides and other products. Prior to the present invention, fermentation of *Myxococcus* has not been conducted for production purposes for any polyketides other than TA and saframycin, which are produced naturally by certain *Myxococcus* strains. Thus, in one aspect, the present invention enables the use of *Myxococcus* as a production host for the production by fermentation of useful bioactive compounds, including, but not limited to, polyketides, non-ribosomal peptides, epothilones, lipases, proteases, other proteins, lipids, glycolipids, rhamnolipids, and polyhydroxyalkanoates.

Among the methods described herein are methods for preparing and storing cell banks and methods for adapting a *Myxococcus* strain to a fermentation medium. These methods are important, because prior to the present invention, frozen cell banks of *Myxococcus* strains adapted for production in oil-based fermentation medium have not been made, and in the absence of adaptation, *Myxococcus* strains frequently die, especially in oil-based fermentation medium.

Also described herein is a fermentation method for growing *Myxococcus* and a fermentation medium useful in the method. Surprisingly, *Myxococcus xanthus* and other *Myxococcus* strains cannot utilize carbohydrates, glycerol, alcohol, or TCA cycle intermediates as a carbon source. Prior to the present invention, M. *xanthus* fermentations were carried out in protein based media. However, NH₄ builds up to levels toxic to growth in protein based media and so limits fermentation. In accordance with the present invention, *Myxococcus* strains may be fermented in a medium that contains oil and/ or fatty acids as a carbon source.

Illustrative oils and fatty acids useful in the method include, but are not limited to, methyl oleate; oils derived from coconut, lard, rapeseed, sesame, soy, and sunflower; salad oil; self emulsifying oils such as Agrimul CoS2, R5O5, and R5O3; glycerol oleate, including glycerol mono oleate and glycerol tri oleate; odd chain esters such as methyl heptadecanoate, methyl nonadecanoate, and methyl pelargonate; ester chains such as propyl oleate and ethyl oleate; vegetable methyl oleate; methyl stearate; methyl linoleate; oleic acid; and phosphatidyl choline, whether pure or derived from soy or egg yolk. Thus, any plant or grain derived oil, such as sunflower or soy oil, any animal derived oil, such as lard oil, free and esterified fatty acids of any chain length both saturated or unsaturated, natural and synthetic fatty acid mixtures such as phosphatidyl choline or methyl pelargonate, respectively, and industrial fermentation oils, such as Cognis Corporation's Agrimul series, can be employed in the method. The fermentation medium may utilize methyl oleate as the carbon source. Generally, oils that are liquid at room temperature are more preferred than solid oils, primarily primarily due to the ease of dispersion. Other important components of the fermentation medium include trace metals such as Fe and Cu, which improve growth and production in complex and defined media and in batch and fed batch processes. A medium containing methyl oleate and trace metals is preferred for the production of epothilones.

Described herein is a fermentation medium for host cells of the invention that contains reduced or no amounts of animal-derived materials. Due to the potential for contamination by infectious agents, such as viruses and prions, the use of animal by-products in fermentation processes for the production of compounds to be administered to humans or animals, one may prefer to use a fermentation medium that contains reduced or no amounts of animal-derived materials. Such media is provided for use in the methods of the invention. The oils or fatty acids contained in the fermentation medium can be derived from a non-animal source, such as a plant. For example, vegetable-derived methyl oleate can be obtained commercially. Moreover, one can replace an animal-derived material with an equivalent but non-identical material derived from a non-animal source. For example, casitone, which is a pancreatic digest of casein, a milk protein, can be replaced with a hydrolysate of a protein from a non-animal source, including but not limited to a plant, such as a vegetable-derived protein hydrolysate.

Generally, fed-batch processes are preferred for fermentation. Feeds force the cells to use nutrients efficiently (for example, the cells metabolize carbon down to CO₂ and H₂O instead of generating toxic organic acids). High nutrient levels can repress secondary metabolism, and if the fermentation feeds nutrients at rate below the threshold of inhibition, production can be higher.

The fermentation methods described herein also include methods related specifically to the production of epothilones and fermentation media useful in those methods. As one example, propionate and acetate can be used to influence the epothilone D:C (or B:A) ratio and the titers of epothilones obtained. While this effect is minimal in the preferred methyl oleate/trace metals fermentation medium, the effect can be quite significant effect in other media, such as CTS medium. Increasing amounts of acetate in the fermentation media can increase *Myxococcus* growth and epothilone production. Acetate alone increases epothilone C (or epothilone A) titers dramatically, and reduces epothilone D (or epothilone A) titers. Propionate alone does not increase epothilone titers and at high concentrations can reduce titers. However, propionate and acetate together can shifts the production from epothilone C (or epothilone A) to epothilone D (or epothilone B). One preferred medium for the production of epothilone D contains casitone, 10 mM acetate, and 30 mM propionate. Media containing odd chain fatty acids can reduce production of epothilone C in fermentations of *Myxococcus xanthus* cells that produce epothilones C and D. Trace metals can also enhance epothilone D production and increase epothilone D:C ratios in the presence of acetate and without any oil in the fermentation media.

Described herein are methods for purifying epothilones from fermentation media and for preparing crystalline forms of epothilone. In general, the purification method involves capture of the epothilone onto XAD resin during fermentation, elution from the resin, solid phase extraction, chromatography, and crystallization. The method is described in detail in Example 3, and while the method is preferred and exemplified for epothilone D, the method can be used to prepare crystalline epothilones generally, including but not limited to other naturally occurring epothilones, and the epothilone analogs produced by the host cells of the invention.

Described herein are novel epothilone derivative compounds in isolated and purified forms useful in agriculture, veterinary practice, and medicine. The compounds may be useful as fungicides, in cancer chemotherapy, as for the prevention of undesired cell growth, including but not limited to the treatement of hyperproliferative diseases such as inflammation, autoimmune disease, and psoriasis, and to the prevention of cell growth in stents. The compound may be an epothilone derivative that is at least as potent against tumor cells as epothilone B or D. The compounds may be useful as immunosuppressants. The compounds may be useful in the manufacture of another compound. The compounds may be formulated in a mixture or solution for administration to a human or animal.

The novel epothilone analogs described herein, as well as the epothilones produced by the host cells of the invention, can be derivatized and formulated as described in PCT patent publication Nos. 93/10121, 97/19086, 98/08849, 98/22461, 98/25929, 99/01124, 99/02514, 99/07692, 99/27890, 99/39694, 99/40047, 99/42602, 99/43320, 99/43653, 99/54318, 99/54319, 99/54330, 99/65913, 99/ 67252, 99/ 67253, and 00/ 00485, and U.S. Patent No. 5,969,145

A detailed description of the invention having been provided above, the following examples are given for the purpose of illustrating the present invention and shall not be construed as being a limitation on the scope of the invention or claims.

### Example 1

### Construction of a Myxococcus xanthus Expression Vector

The DNA providing the integration and attachment function of phage Mx8 was inserted into commercially available pACYC184 (New England Biolabs). An ~2360 bp *Mfe*I*-Sma*I from plasmid pPLH343, described in Salmi *et al.,* Feb. 1998, *J. Bact. 180*(3): 614-621, was isolated and ligated to the large-*Eco*RI-*Xmn*I restriction fragment of plasmid pACYC184. The circular DNA thus formed was -6 kb in size and called plasmid pKOS35-77.

Plasmid pKOS35-77 serves as a convenient plasmid for expressing recombinant PKS genes of the invention under the control of the epothilone PKS gene promoter. In one illustrative embodiment, the entire epothilone PKS gene with its homologous promoter is inserted in one or more fragments into the plasmid to yield an expression vector of the invention.

Expression vectors may be provided in which the recombinant PKS genes are under the control of a *Myxococcus xanthus* promoter. To construct an illustrative vector, the promoter of the *pilA* gene of M. *xanthus* was isolated as a PCR amplification product. Plasmid pSWU357, which comprises the *pilA* gene promoter and is described in Wu and Kaiser, Dec. 1997, *J. Bact.* 179(24):7748-7758, was mixed with PCR primers Seql and Mxpil1 primers: Seq1: 5'-AGCGGATAACAATTTCACACAGGAAACAGC-3'; and Mxpil1: 5'-TTAATTAAGAGAAGGTTGCAACGGGGGC-3', and amplified using standard PCR conditions to yield an ~800 bp fragment. This fragment was cleaved with restriction enzyme *Kpnl* and ligated to the large *KpnI-EcoRV* restriction fragment of commercially available plasmid pLitmus 28 (New England Biolabs). The resulting circular DNA was designated plasmid pKOS35-71B.

The promoter of the *pilA* gene from plasmid pKOS35-71B was isolated as an ~800 bp *Eco*RV-*Sna*BI restriction fragment and ligated with the large *MscI* restriction fragment of plasmid pKOS35-77 to yield a circular DNA -6.8 kb in size. Because the ~800 bp fragment could be inserted in either one of two orientations, the ligation produced two plasmids of the same size, which were designated as plasmids pKOS35-82.1 and pKOS35-822 Restriction site and function maps of these plasmids are presented in Figure 2.

Plasmids pKOS35-82.1 and pKOS35-822 serve as convenient starting materials for vectors in which a recombinant PKS gene is placed under the control of the *Myxococcus xanthus pilA* gene promoter. These plasmids comprise a single *PacI* restriction enzyme recognition sequence placed immediately downstream of the transcription start site of the promoter. In one illustrative embodiment, the entire epothilone PKS gene without its homologous promoter is inserted in one or more fragments into the plasmids at the *PacI* site to yield expression vectors .

The sequence of the *pilA* promoter in these plasmids is shown below.

CGACGCAGGTGAAGCTGCTTCGTGTGCTCCAGGAGCGGAAGGTGAAGCCG
GTCGGCAGCGCCGCGGAGATTCCCTTCCAGGCGCGTGTCATCGCGGCAAC
GAACCGGCGGCTCGAAGCCGAAGTAAAGGCCGGACGCTTTCGTGAGGACC
TCTTCTACCGGCTCAACGTCATCACGTTGGAGCTGCCTCCACTGCGCGAGC
GTTCCGGCGACGTGTCGTTGCTGGCGAACTACTTCCTGTCCAGACTGTCGG
AGGAGTTGGGGCGACCCGGTCTGCGTTTCTCCCCCGAGACACTGGGGCTAT
TGGAGCGCTATCCCTTCCCAGGCAACGTGCGGCAGCTGCAGAACATGGTG
GAGCGGGCCGCGACCCTGTCGGATTCAGACCTCCTGGGGCCCTCCACGCTT
CCACCCGCAGTGCGGGGCGATACAGACCCCGCCGTGCGTCCCGTGGAGGG
CAGTGAGCCAGGGCTGGTGGCGGGCTTCAACCTGGAGCGGCATCTCGACG
ACAGCGAGCGGCGCTATCTCGTCGCGGCGATGAAGCAGGCCGGGGGCGTG
AAGACCCGTGCTGCGGAGTTGCTGGGCCTTTCGTTCCGTTCATTCCGCTACC
GGTTGGCCAAGCATGGGCTGACGGATGACTTGGAGCCCGGGAGCGCTTCG
GATGCGTAGGCTGATCGACAGTTATCGTCAGCGTCACTGCCGAATTTTGTC
AGCCCTGGACCCATCCTCGCCGAGGGGATTGTTCCAAGCCTTGAGAATTGG
GGGGCTTGGAGTGCGCACCTGGGTTGGCATGCGTAGTGCTAATCCCATCCG
CGGGCGCAGTGCCCCCCGTTGCAACCTTCTCTTAATTAA

To make recombinant *Myxococcus xanthus* host cells, *M. xanthus* cells are grown in CYE media (Campos and Zusman, 1975, Regulation of development in *Myxococcus xanthus*: effect of 3': 5'-cyclic AMP, ADP, and nutrition, *Proc. Natl. Acad. Sci. USA* 72: 518-522) to a Klett of 100 at 30°C at 300 rpm. The remainder of the protocol is conducted at 25°C unless otherwise indicated. The cells are then pelleted by centrifugation (8000 rpm for 10 min. in an SS34 or SA600 rotor) and resuspended in deionized water. The cells are again pelleted and resuspended in 1/100th of the original volume.

DNA (one to two µL) is electroporated into the cells in a 0.1 cm cuvette at room temperature at 400 ohm, 25 µFD, 0.65 V with a time constant in the range of 8.8 - 9.4. The DNA is free of salts and is resuspended in distilled and deionized water or dialyzed on a 0.025 µm Type VS membrane (Millipore). For low efficiency electroporations, the DNA is spot dialyzed, and outgrowth is in CYE. Immediately after electroporation, 1 mL of CYE is added, and the cells in the cuvette pooled with an additional 1.5 mL of CYE previously added to a 50 mL Erlenmeyer flask (total volume 2.5 ml). The cells are grown for four to eight hours (or overnight) at 30 to 32°C at 300 rpm to allow for expression of the selectable marker. Then, the cells are plated in CYE soft agar on plates with selection. With kanamycin as the selectable marker, typical yields are 10³ to 10⁵ per µg of DNA. With streptomycin as the selectable marker, it is included in the top agar, because it binds agar.

With this procedure, the recombinant DNA expression vectors are electroporated into *Myxococcus* host cells that express recombinant PKSs and produce the epothilone, epothilone derivatives, and other novel polyketides encoded thereby.

### Example 2

### Chromosomal Integration and a Bacterial Artificial Chromosome (BAC) for Expression of Epothilone in Myxococcus xanthus

To express the epothilone PKS and modification enzyme genes in a heterologous host to produce epothilones by fermentation, *Myxococcus xanthus,* which is closely related to *Sorangium cellulosum* and for which a number of cloning vectors are available, is employed in accordance with the methods described herein. *M. xanthus* and *S. cellulosum* are myxobacteria and so may share common elements of gene expression, translational control, and post translational modification. *M. xanthus* has been developed for gene cloning and expression: DNA can be introduced by electroporation, and a number of vectors and genetic markers are available for the introduction of foreign DNA, including those that permit its stable insertion into the chromosome. *M. xanthus* can be grown with relative ease in complex media in fermentors and can be subjected to manipulations to increase gene expression, if required.

To introduce the epothilone gene cluster into *Myxococcus xanthus,* one can build the epothilone cluster into the chromosome by using homologous recombination to assemble the complete gene cluster. Alternatively, the complete epothilone gene cluster can be cloned on a bacterial artificial chromosome (BAC) and then moved into M. *xanthus* for integration into the chromosome..

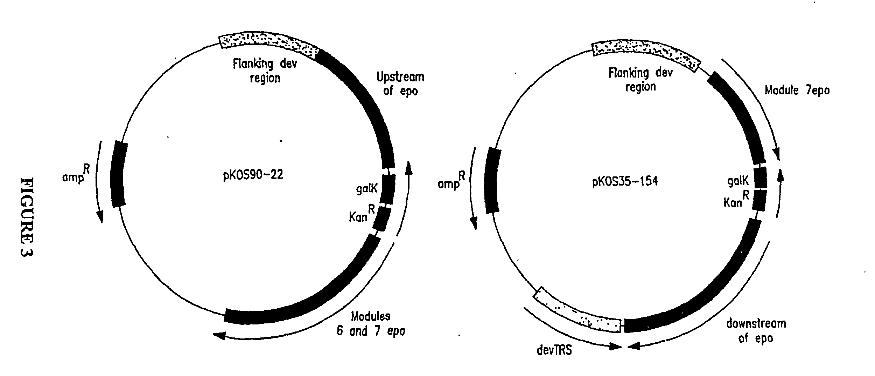

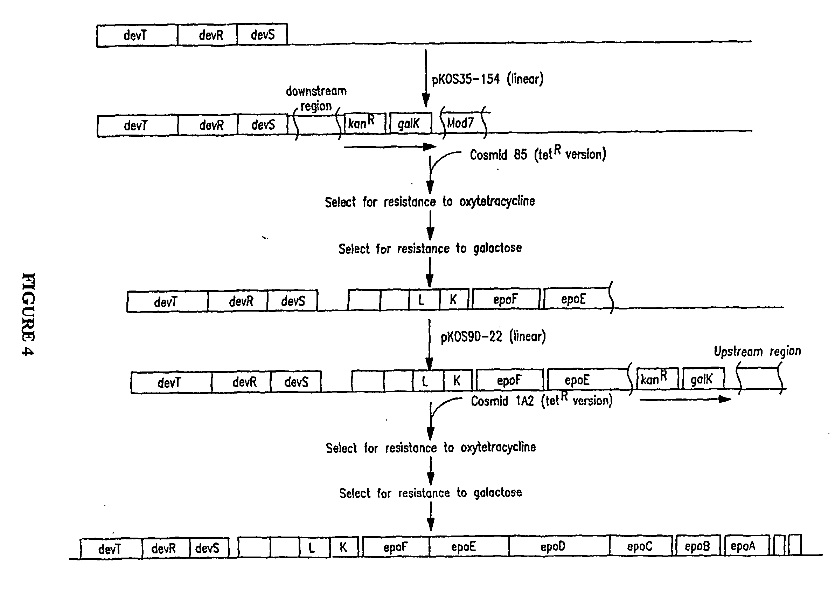

To assemble the gene cluster from cosmids pKOS35-70.1A2, and pKOS35-79.85, small regions (~2 kb or larger) of homology from these cosmids are introduced into *Myxococcus xanthus* to provide recombination sites for larger pieces of the gene cluster: As shown in Figure 3, plasmids pKOS35-154 and pKOS90-22 are created to introduce these recombination sites. The strategy for assembling the epothilone gene cluster in the M. *xanthus* chromosome is shown in Figure 4. Initially, a neutral site in the bacterial chromosome is chosen that does not disrupt any genes or transcriptional units. One such region is downstream of the *devS* gene, which has been shown not to affect the growth or development of M. *xantluts.* The first plasmid, pKOS35-154, is linearized with *Dra*I and electroporated into M. *xanthus*. This plasmid contains two regions of the *dev* locus flanking two fragments of the epothilone gene cluster. Inserted in between the epo gene regions is a cassette composed of a kanamycin resistance marker and the E. *coli galK* gene. See Ueki *et al*., 1996, *Gene* 183: 153-157. Kanamycin resistance arises in colonies if the DNA recombines into the *dev* region by a double recombination using the *dev* sequence as regions of homology.

This strain, K35-159, contains small (~2.5 kb) regions of the epothilone gene cluster that will allow for recombination of pKOS35-79.85. Because the resistance markers on pKOS35-79.85 are the same as that in K35-159, a tetracycline transposon was transposed into the cosmid, and cosmids that contain the transposon inserted into the kanamycin marker were selected. This cosmid, pKOS90-23, was electroporated into K35-159, and oxytetracycline resistant colonies were selected to create strain K35-174. To remove the unwanted regions from the cosmid and leave only the epothilone genes, cells were plated on CYE plates containing 1 % galactose. The presence of the *galK* gene makes the cells sensitive to 1% galactose. Galactose resistant colonies of K35-174 represent cells that have lost the *galK* marker by recombination or by a mutation in the *galK* gene. If the recombination event occurs, then the galactose resistant strain is sensitive to kanamycin and oxytetracycline. Strains sensitive to both antibiotics are verified by Southern blot analysis. The correct strain is identified and designated K35-175 and contains the epothilone gene cluster from module 7 to 4680 bp downstream of the stop codon of *epoK.*

To introduce modules 1 through module 7, the above process is repeated once more. The plasmid pKOS90-22 is linearized with *Dra*I and electroporated into K35-175 to create K111-13.2. This strain is electroporated with the tetracycline resistant version of pKOS35-70.1A2, pKOS90-38, and colonies resistant to oxytetracycline are selected. This creates strain K111-13.23. Recombinants that now have the whole epothilone gene cluster are selected by resistance to 1% galactose. This results in clones K111-32.25, K111-32.26, and K111-32.35. Strain K111-32.25 was deposited April 14, 2000, with the American Type Culture Collection, Manassas, VA 20110-2209, USA, in compliance with the Budapest Treaty and is available under accession No. PTA-1700. This strain contains all the epothilone genes and their promoter(s).

Fermentation was performed by inoculating strains into 5 mL of CYE (10 g casitone, 5 g yeast extract, and 1 g MgSO₄·7H₂O per liter) in a 50 mL flask and growing overnight until the culture was in mid log growth phase. A 100 µL aliquot was spread onto a CTS plate, and the plate incubated at 32°C for 4 to 5 days. To extract epothilones, the agar and cells from the plate was macerated, put in a 50 mL conical tube, and acetone added to fill the tube. The solution was incubated with rocking for 4 to 5 hours, the acetone evaporated, and the remaining liquid extracted twice with an equal volume of ethyl acetate. The water was removed from the ethyl acetate extract by adding magnesium sulfate. The magnesium sulfate was filtered out, and the liquid was evaporated to dryness. The epothilones were resuspended in 200 µL of acetonitrile and analyzed by LC/MS. The analysis showed that the strain produced epothilones A and B, with epothilone B present at about 0.1 mg/L in the culture, and epothilone A at 5 to 10-fold lower levels.

This strain can also be used to produce epothilones in liquid culture. A flask containing CYE is inoculated with an epothilone producing strain. The next day, while the cells are in mid-log growth phase, a 5% inoculum is added to a flask containing 0.5% CMM (0.5% casitone, 0.2% MgSO₄•7H₂O, 10 mM MOPS pH7.6) along with 1 mg/mL serine, alanine, and glycine and 0.1% sodium puyruvate. The sodium pyruvate can be added to 0.5% to increase epothilone B production but causes a decrease in the ratio of epothilone B to epothilone A. The culture is grown at 30°C for 60-72 hours. Longer incubations result in a decrease in titers of epothilones. To recover epothilones, the cultures are centrifuged at 10,000 rpm for 10 minutes in an SS34 rotor. The supernatants are extracted twice with ethyl acetate and rotary evaporated ("rotavaped") to dryness. Liquid cultures produced 2 to 3 mg/L of epothilones A and B, with ratios similar to that observed with plate cultures. If XAD (0.5 - 2%) was added to the culture, epothilones C and D were observed, with epothilone D present at 0.1 mg/L and epothilone C present at 5 to 10-fold lower levels.

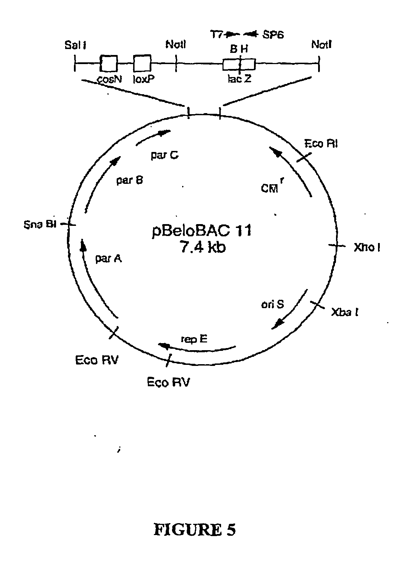

To clone the whole gene cluster as one fragment, a bacterial artifical chromosome (BAC) library is constructed. First, SMP44 cells are embedded in agarose and lysed according to the BIO-RAD genomic DNA plug kit. DNA plugs are partially digested with restriction enzyme, such as *Sau*3AI or *Hin*dIII, and electrophoresed on a FIGE or CHEF gel. DNA fragments are isolated by electroeluting the DNA from the agarose or using gelase to degrade the agarose. The method of choice to isolate the fragments is electroelution, as described in Strong *et al*., 1997, *Nucleic Acids Res. 19*: 3959-3961. The DNA is ligated into the BAC (pBeloBACII) cleaved with the appropriate enzyme. A map of pBeloBACII is shown in Figure 5.

The DNA is electroporated into DH10B cells by the method of Sheng *et al., 1995, Nucleic Acids Res. 23*:1990-1996 to create a *Sorangium cellulosum* genomic library. Colonies are screened using a probe from the NRPS region of the epothilone cluster. Positive clones are picked and DNA is isolated for restriction analysis to confirm the presence of the complete gene cluster. This positive clone is designated pKOS35-178.

To create a strain that can be used to introduce pKOS35-178, a plasmid, pKOS35-164, is constructed that contains regions of homology that are upstream and downstream of the epothilone gene cluster flanked by the *dev* locus and containing the kanamycin resistance *galK* cassette, analogous to plasmids pKOS90-22 and pKOS35-154. This plasmid is linearized with *Dra*I and electroporated into Myxococcus xanthus, in accordance with the method of Kafeshi et *al*., 1995, *Mol. Microbiol. 15*: 483-494, to create K35-183. The plasmid pKOS35-178 can be introduced into K35-183 by electroporation or by transduction with bacteriophage P1, and chloramphenicol resistant colonies are selected. Alternatively, a version of pKOS35-178 that contains the origin of conjugative transfer from pRP4 can be constructed for transfer of DNA from *E. coli* to K35-183. This plasmid is made by first constructing a transposon containing the oriT region from RP4 and the tetracycline resistance maker from pACYC184 and then transposing the transposon *in vitro* or *in vivo* onto pKOS35-178. This plasmid is transformed into S17-1 and conjugated into *M. xanthus*. This strain, K35-190, is grown in the presence of 1% galactose to select for the second recombination event. This strain contains all the epothilone genes as well as all potential promoters. This strain is fermented and tested for the production of epothilones A and B.

Alternatively, the transposon can be recombined into the BAC using either the temperature sensitive plasmid pMAK705 or pKO3 by transposing the transposon onto either pMAK705 or pKO3, selecting for tetR and camS plasmids; the recombination is accomplished as described in Hamilton *et al*., Sep. 1989, *J. Bact. 171*(9): 4617-4622 and Link *et al*., Oct. 1997, *J. Bact*. *179*(20): 6228-6237.

Besides integrating pKOS35-178 into the *dev* locus, it can also be integrated into a phage attachment site using integration functions from myxophages Mx8 or Mx9. A transposon is constructed that contains the integration genes and *att* site from either Mx8 or Mx9 along with the tetracycline gene from pACYC184. Alternative versions of this transposon may have.only the attachment site. In this version, the integration genes are then supplied in trans by coelectroporation of a plasmid containing the integrase gene or having the integrase protein expressed in the electroporated strain from any constitutive promoter, such as the *mgl* promoter (see Magrini *et al*., Jul. 1999, *J. Bact*. *181*(13): 4062-4070, incorporated herein by reference). Once the transposon is constructed, it is transposed onto pKOS35-178 to create pKOS35-191. This plasmid is introduced into *Myxococcus xanthus* as described above. This strain contains all the epothilone genes as well as all potential promoters. This strain is fermented and tested for the production of epothilones A and B. Alternatively, a strain that contains the *att* site and the *oriT* region can be transposed onto the BAC and the resulting BAC conjugated into *M. xanthus*.

Once the epothilone genes have been established in a strain of *Myxococcus xanthus,* manipulation of any part of the gene cluster, such as changing promoters or swapping modules, can be performed using the kanamycin resistance and *galK* cassette, as described below. Cultures *of Myxococcus xanthus* containing the *epo* genes are grown in a number of media and examined for production of . epothilones. If the levels of production of epothilones (in particular B or D) are low, then the M. *xanthus*-producing clones are subjected to media development and mutation based strain improvement, as described in the following example.

### Example 3

### Processes for the Production and Purification of Epothilones

### A. Optimizing the Heterologous Production of Epothilone D in Mvxococcus xanthus

The heterologous production of epothilone D in *Myxococcus xanthus* was improved by 140-fold from an initial titer of 0.16 mg/L with the incorporation of an adsorber resin, the identification of a suitable carbon source, and the implementation of a fed-batch process.

To reduce the degradation of epothilone D in the basal medium, XAD-16 (20 g/L) was added to stabilize the extracellular product. This greatly facilitated its recovery and enhanced the yield by three-fold. The use of oils as a carbon source for cell growth and product formation was also evaluated. From a screen of various oils, methyl oleate was shown to have the greatest impact. At the optimal concentration of 7 mL/L in a batch process, the maximum cell density was increased from 0.4 g dry cell weight (DCW)/L to 2 g DCW/L. Product yield depended on the presence of trace elements in the production medium. With an exogenous supplement of trace metals to the basal medium, the peak epothilone D titer was enhanced eight-fold, demonstrating the significant role of metal ions in cell metabolism and in epothilone biosynthesis. To increase the product yield further, a continuous fed-batch process was employed to promote a higher cell density and to maintain an extended production period. The optimized fed-batch cultures consistently yielded a cell density of 7 g DCW/L and an average production titer of 23 mg/L.

Epothilones are secondary metabolites that are naturally produced by various strains of the myxobacterium *Sorangium cellulosum* (Gerth *et al*., 1996; Gerth *et al*., 2001; references cited in this example are listed at the end of this section). They are potent inhibitors of microtubule depolymerization, with a mechanism of action similar to that of the anti-cancer drug Taxol (Bollag et *al*., 1995). Their cytotoxic effect against multiple-drug resistant tumor cell lines expressing the P-glycoprotein renders them potential therapeutic compounds with great commercial value (Su *et al*., 1997; Kowalski et *al*., 1997). Their comparatively high solubility in water also facilitates their formulation for clinical evaluation.

Epothilones A and B are the major fermentation products of the natural host (Gerth *et al*., 1996). The macrocyclic core of these polyketide molecules is formed by the successive decarboxylative condensations of acetate and propionate units (Gerth *et al*., 2000). Epothilones A and B differ by a single methyl group at the C-12 position of their carbon skeleton. This structural variance results from the incorporation of an acetate in the assembly of epothilone A and a propionate in that of epothilone B. Epothilones C and D are intermediates in the biosynthetic pathway of epothilones A and B, respectively (Tang *et al*., 2000; Molnár *et al*., 2000). They are excreted as minor products during the fermentation process, with a combined yield of about 0.4 mg/L. Because preliminary *in vivo* studies revealed epothilone D to be the most promising of the four compounds as an anti-tumor drug (Chou *et al*., 1998), it is of considerable interest to produce this molecule on a large scale.

The gene cluster responsible for the biosynthesis of the epothilones has been sequenced (Tang et *al*., 2000; Molnar *et al.,* 2000) and used to produce these compounds in *Myxococcus xanthus*, a microbial host closely-related to S. *cellulosum* but more amenable to genetic manipulation. To foster the production of epothilone D, a deletion mutant of this recombinant strain (described in Example 4, below) was constructed to inactivate the P450 epoxidase that catalyzes the conversion of epothilones C and D to epothilones A and B, respectively (Tang *et al*., 2000). This genetic alteration effectively promoted the secretion of epothilones C and D as sole products of the *M. xanthus* fermentation, with an epothilone D to C ratio of 4 to 1. The resulting mutant offers a distinct advantage over the natural host in the recovery and purification of the desired product. In this example, improvements in media composition and fermentation strategy are described that result in a 140-fold increase in the production of epothilone D in M. *xanthus.*

Adsorber resins have been used in the fermentations of myxobacteria for the continuous capture of biologically active molecules produced at low quantities (Reichenbach and Höfle, 1993). To facilitate the isolation of epothilone D, the hydrophobic resin XAD-16 was added to the culture medium. Because the bound product can readily be eluted from the resin with an appropriate solvent, its recovery was greatly simplified. Moreover, the use of XAD-16 minimized epothilone degradation through product stabilization.

*Myxococcus xanthus* has been traditionally cultivated in media consisting primarily of enzymatic hydrolysates of casein, such as peptone and casitone, relying on amino acids as the sole carbon and nitrogen source (Reichenbach and Dworkin, 1991). Consequently, ammonia is accumulated in the fermentation broth as a result of amino acid degradation. It was demonstrated by Gerth *et al.* (1986) that an extracellular ammonia concentration of 35-42 mM in a *Myxococcus virescens* culture corresponded to a surprisingly high ammonia concentration of 80-140 mM within the cells. More importantly, it was shown that by continuously removing the excess ammonia to below 8 mM with an in situ membrane process, both cell mass and secondary metabolite production dramatically increased (Hecht et *al*., 1990). Because the generation of high levels of ammonia is speculated to be inhibitory to the growth of M. *xanthus* and epothilone D production, an alternative carbon source to reduce the consumption of amino acids is desirable.

Although an adaptation process was required, methyl oleate was identified from an extensive screen of different oils as a substrate that can be metabolized by *M. xanthus;* With the addition of an exogenous trace element solution to the growth medium, epothilone D production was enhanced 8-fold, with a yield of 3.3 mg/L in a simple batch fermentation. To optimize the process further, a fed-batch approach using intermittent or continuous feeds of casitone and methyl oleate was adopted to prolong the production phase of the cells. A comparison of the results obtained with the two different feed strategies is reported in this example..

### MATERIALS AND METHODS

### Inoculum Preparation

For the production of epothilone D in culture media without methyl oleate, 1 mL of frozen cells of the *Myxococcus xanthus* strain K111-40.1 in 20 % (v/v) glycerol was inoculated into 3 mL of CYE medium consisting of 10 g/L casitone (Difco), 5 g/L yeast extract (Difco), 1 g/L MgSO₄·7H₂O, and 50 mM HEPES, pri 7.6, in a 50-mL glass culture tube. The HEPES buffer solution was titrated to pH 7.6 with potassium hydroxide. The cells were incubated at 30°C and 175 rpm on a rotary shaker for 3 days. They were then transferred to a 250-mL Erlenmeyer flask containing 50 mL of CYE medium and grown for 2 days under the same conditions. The resulting seed culture was used to inoculate 50-mL production flasks at an inoculum size of 5% (v/v).

For the cultivation of M. *xanthus* in media containing methyl oleate, the cells had to be adapted to growth in the presence of the oil. One seed vial of frozen cells was inoculated into 3 mL of CYE medium that was supplemented with 3 µL of methyl oleate (Emerest 2301) (Cognis Corp.). The cells were grown in a glass culture tube for 2-6 days at 30°C and 175 rpm until the culture was sufficiently dense under a microscope. They were then transferred into a 250 mL Erlenmeyer flask containing 50 mL of CYE-MOM medium consisting of 10 g/L casitone (Difco), 5 g/L yeast extract (Difco), 1 g/L MgSO₄·7H₂O, 2 mL/L methyl oleate, and 50 mM HEPES, pH 7.6. After 2 days of growth, the cells were frozen and stored at -80°C as 1 mL aliquots in 20% (v/v) glycerol.

For the production of epothilone D in media containing methyl oleate, 1 mL of the frozen oil-adapted cells was inoculated into 3 mL of CYE-MOM medium in a glass culture tube. The cells were incubated at 30°C and 175 rpm for 2 days and transferred to a 250 mL Erlenmeyer flask containing 50 mL of CYE-MOM medium. The resulting seed culture was grown for 2 days under the same conditions and was used to inoculate 50 mL production flasks at an inoculum size of 5% (v/v).

In preparing the inoculum for 5-L fermentations, 25 mL of the oil-adapted seed culture were transferred into a 2.8 L Fernbach flask containing 475 mL of CYE-MOM medium. The cells were grown at 30°C and 175 rpm for 2 days. Subsequently, 250 mL of this secondary seed culture was inoculated into 5-L fermentors containing 4.75 L of production medium to yield a final inoculum concentration of 5% (v/v).

### Shake Flask Production

Batch cultivations of *M. xanthus* K111-40-1 in the absence of methyl oleate were prepared as follows. One gram of XAD-16 resin (Rohm and Haas) was autoclaved at 121°C for 30 min in a 250-mL Erlenmeyer flask with 5 mL of deionized water. The excess water was then removed from the flask, and 50 mL of CTS medium consisting of 5 g/L casitone, 2 g/L MgSO₄·7H₂O, and 50 mM HEPES, pH 7.6, were added. Because autoclaving of the adsorber resin in the presence of the production medium led to the binding of essential nutrients required by the cells, the resin and medium components were sterilized separately. The production flasks were inoculated with 2.5 mL of seed culture and incubated at 30°C and 175 rpm for 6 days.

Batch cultivations in the presence of methyl oleate were prepared as described above. In addition, the production medium was supplemented with 7 mL/L of methyl oleate and 4 mL/L of a filter-sterilized trace element solution that was composed of 10 mL/L concentrated H₂SO₄, 14.6 g/L FeCl₃·6H₂O, 2.0 g/L ZnCl₃, 3.0 g/L MnCl₂·4H₂O, 0.43 g/L CuCl₂·2H₂O, 0.31 g/L H₃B0₃, 0.24 g/L CaCl₂·6H₂O, and 0.24 g/L Na₂MO₄·2H₂O. The production flasks were then inoculated with 2.5 mL of the oil-adapted seed culture and grown at 30°C and 175 rpm for 5 days.

Fed-batch cultures with intermittent feeds of casitone and methyl oleate were prepared as follows. One gram of XAD-16 resin was autoclaved at 121°C for 30 min. in a 250 mL Erlenmeyer flask with 5 mL of deionized water. After sterilization, the excess water was removed from the flask, and 50 mL of CTS medium supplemented with 2 mL/L methyl oleate, 4 mL/L trace element solution, and 50 mM HEPES, pH 7.6, were added. The production flasks were inoculated with 25 mL of the oil-adapted seed culture and incubated at 30°C and 175 rpm. Two days after inoculation, 2 g/L of casitone and 3 mL/L of methyl oleate were added to the culture medium at 24 h intervals. The casitone feed was prepared as a concentrated 100 g/L solution. The cultures were grown for 10-12 days until substantial cell lysis was observed.

All the production cultures can be grown on a 500-mL scale in 2.8-L Fernbach flasks under the same growth conditions.

### Fementor Production

Fed-batch fermentations, on a 5-L scale with intermittent or continuous feeds of casitone and methyl oleate were prepared as follows. Twenty grams per liter of XAD-16 and 2 g/L of MgSO₄·7H₂O was autoclaved at 121°C for 30 min in a 5-L fermentor (B. Braun) with 4.75 L of deionized water. After sterilization, a concentrated casitone solution (150 g/L), methyl oleate, and trace elements were added to the bioreactor aseptically to attain a final casitone, methyl oleate, and trace element concentration of 5 g/L, 2 mL/L, and 4 mL/L, respectively. The medium was then inoculated with 250 mL of the oil-adapted seed culture. The fermentation was performed at 30°C with an aeration rate of 0.4-0.5 v/v/m and an initial agitation rate of 400 rpm. The dissolved oxygen was controlled at 50% of saturation by a stirring cascade between 400-700 rpm. Cultivation pH was maintained at 7.4 by the automated addition of 2.5 N KOH and 25 MH₂SO₄. Twenty-four hours after inoculation, casitone (150 g/L) and methyl oleate were added to the production medium at a feed rate of 2 g/L/ day casitone and 3 mL/L/ day methyl oleate. The feeds were delivered either as a single bolus every 24 h or continuously with peristaltic pumps (W. Marlow). The cells were allowed to grow for 10-12 days until considerable cell lysis was noted.

### Epothilone Quantitation

Prior to the use of the XAD-16 resin in the fermentations, 1 mL of culture broth was sampled from the production flasks or bioreactors and centrifuged at 13,000 g for 10 min. Quantitation of the epothilone products in the supernatant was carried out using a Hewlett Packard 1090 HPLC with UV detection at 250 nm. Five hundred microliters of the supernatant were injected across a 4.6 x 10 mm guard column (Inertsil, ODS-3, 5 µm). An online extraction was then performed at a flow rate of 1 mL/min. with a 100% water wash for 0.5 min., followed by a gradient to 50% acetonitrile over 1.5 min. The eluant was diverted to waste for the first two minutes and was passed onto a longer separation column (4.6 x 150 mm, Inertsil, ODS-3, 5 µm) thereafter. Separation of epothilones C and D was performed with a gradient from 50% to 100% acetonitrile over 8 min, followed by a 100% acetonitrile wash for 3 min. Under these conditions, epothilone C eluted at 9.4 minutes and epothilone D eluted at 9.8 minutes.

With the use of the XAD-16 resin, 5-50 mL of well-mixed culture broth and resin were sampled from the production flasks or bioreactors. After the resin was settled by gravity, the culture broth was decanted. The resin was washed with 5-50 mL of water and allowed to settle by gravity again. The aqueous mixture was completely removed, and the epothilone products were extracted from the resin with 100% methanol. The amount of solvent used was equivalent to 50% of the sample volume. Quantitation of epothilones C and D was carried out by HPLC analysis with UV detection at 250 nm. Fifty microliters of the methanol extract were injected across two 4.6 x 10 mm guard columns (Inertsil, ODS-3,5 µm) and a longer 4.6 x 150 mm column (Inertsil, ODS-3, 5 µm). The assay method was isocratic, eluting with 60% acetonitrile and 40% water for 18 min at a flow rate of 1 mL/min. Under these conditions, epothilone C was detected at 10.3 minutes and epothilone D was detected at 13.0 minutes. Standards were prepared using purified epothilone D.

### Cell Growth Determination

Cell growth in the absence of methyl oleate was monitored by measuring the optical density (OD) at 600 nm. Samples were diluted with water until the final OD values were less than 0.4. Because the addition of methyl oleate to culture medium results in the formation of an emulsion that has a strong absorbance at 600 nm, cell growth in the presence of methyl oleate was determined by dry cell weight (DCW). Forty milliliters of culture broth were centrifuged at 4200 g for 20 min in preweighed test tubes. The pellets were then washed with 40 mL of water and dried for 2 days at 80°C before weighing.

### Ammonia Determination

One milliliter of fermentation broth was clarified by centrifugation at 13,000 g for 5 man. The supernatant was then used for ammonia analysis with an ammonia assay kit (Sigma). Samples were diluted 20-100 fold with water until the final concentrations were less than 880 µM.

### Methyl Oleate Determination

The residual methyl oleate in 1-mL of fermentation broth was extracted with 5 mL of acetonitrile. The mixture was vortexed and clarified by centrifugation at 4200 g for 20 min. Quantitation of methyl oleate was carried out by HPLC analysis with UV detection at 210 nm. Fifty microliters of the supernatant were injected across two 4.6 x 10 mm guard columns (Inertsil, ODS-3, 5 µm) and a longer 4.6 x 150 mm column (Inertsil, ODS-3, 5 µm). The column was washed with acetonitrile-water (1:1) for 2 min. at a flow rate of 1 mL/min. It was then eluted with a gradient of 50% to 100% acetonitrile over 24 min., followed by a 100% acetonitrile wash for 5 min. Because of the heterogeneity of the carbon chain lengths of commercial methyl oleate, this compound was eluted as two main peaks that were detected at 25.3 minutes and 27.1 minutes. Methyl oleate bound to the XAD-16 resin was quantitated from the methanol extract using the same HPLC method. Standards of methyl oleate were prepared in 83.3% acetonitrile. Consumption of methyl oleate by the cells was calculated as: (total methyl oleate added) - (residual methyl oleate in medium) - (methyl oleate bound to resin).

### RESULTS

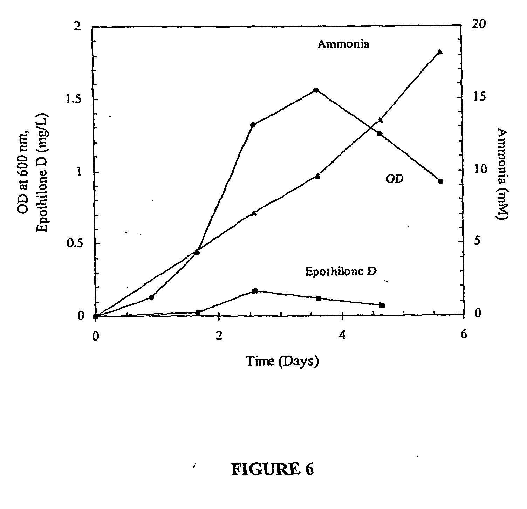

The *Myxococcus xanthus* strain K111-40-1 was initially cultivated in a batch fermentation process with a simple production medium consisting only of 5 g/L casitone (a pancreatic casein digest) and 2 g/L magnesium sulfate. The baseline performance of the cells is shown in Figure 6.

Maximum cell density and epothilone D production were attained three days after inoculation at an OD₆₀₀ of 1.6 and a corresponding titer of 0.16 mg/L. Both cell density and product yield decreased substantially thereafter. With the consumption of casitone by the cells, a gradual accumulation of ammonium was also detected in the production medium. The final ammonia concentration approached 20 mM at the end of the 5-day fermentation.

### Effect of XAD-16 on product stability

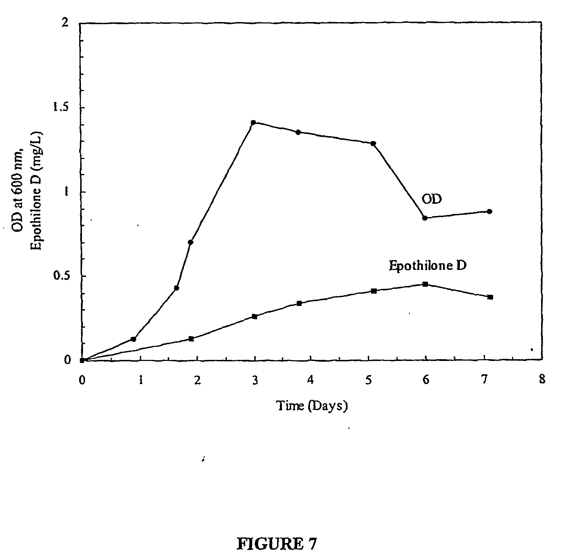

To prevent the rapid degradation of epothilone D, the hydrophobic adsorber resin, XAD-16, was added to the production medium to bind and stabilize the excreted product XAD-16 is a polyaromatic resin that had previously been used by Gerth *et al.* (1996) for the isolation of epothilones A and B from fermentations of the microbial producer, *Sorangium cellulosum* So ce90. As shown in Figure 7, the presence of the adsorber resin did not affect the growth of the cells. However, it effectively reduced the loss of epothilone D in the fermentation broth, which led to a three-fold enhancement in the recovery of this product

### Media Development

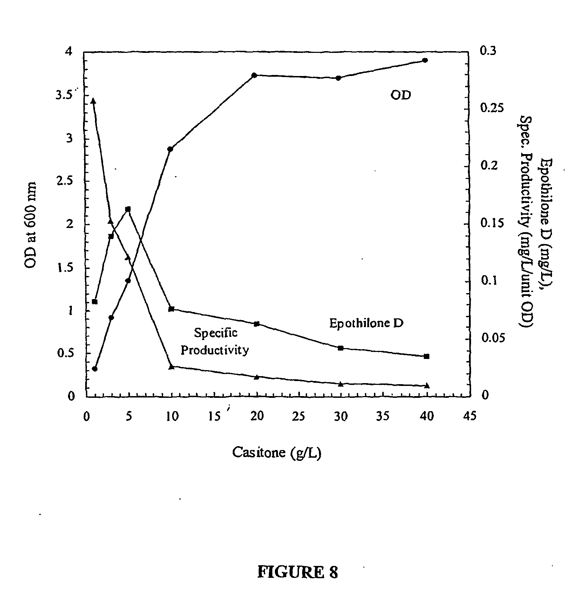

In an effort to develop a medium that can support higher cell density and epothilone production, the influence of casitone on growth and product yield was evaluated by varying its concentration from 1 g/L to 40 g/L in the production medium. Although cell growth was stimulated with increasing casitone concentrations, the specific productivity of the cells declined significantly, as shown in Figure 8. The optimal casitone concentration for epothilone D production was reached at 5 g/L, with higher concentrations resulting in decreased titers.

Because media improvements were limited with the use of casitone, alternative substrates were evaluated as supplements to the basal production medium. From a detailed screen of different oils, methyl oleate was identified as a carbon source that promoted the greatest increase in epothilone D production, as summarized in Table 3.

**TABLE 3**

| **Oil (7 mL/L)** | **Epothilone D production relative to control with no oil supplements (%)** |
|---|---|
| Methyl Oleate | 780 |
| Ethyl Oleate | 740 |
| Coconut Oil | 610 |
| Lard | 470 |
| Propyl Oleate | 420 |
| Sesame Oil | 380 |
| Glycerol Tri-oleate | 370 |
| Salad Oil | 360 |
| Sunflower Oil | 330 |
| Soy Oil | 290 |
| Methyl Heptadecanoate | 190 |
| No Oil (Control) | 100 |
| Methyl Nonadecanoate | 96 |
| Methyl Pelargoante | 40 |
| Rapeseed Oil | 40 |

However, the direct addition of methyl oleate to the production medium resulted in premature cell lysis. Therefore, the seed cultures were grown in the presence of methyl oleate prior to the production fermentations. Interestingly, this adaptation process rendered the cells less susceptible to lysis. As shown in Table 4 (Improvements in Growth and Production Compared to Baseline Performance in CTS Medium in Batch Fermentation), a peak biomass concentration of 2.1 g/L and an epothilone D titer of 3.3 mg/L were achieved with a methyl oleate concentration of 7 mL/L and a trace elements concentration of 4 mL/L.

**TABLE 4**

| **Fermentation Conditions** | **Maximum Cell Density (g DCW/L)** | **Maximum Epothilone D Production (mg/L)** |
|---|---|---|
| CTS medium with no XAD-16 in batch process | 0.44 ± 0.04 | 0.16 ± 0.03 |
| CTS medium with XAD-16 in batch process | 0.44 ± 0.04 | 0.45 ± 0.09 |
| CTS medium with 7 mL/L methyl oleate in batch process | 1.2 ± 0.1 | 0.12 ± 0.02 |
| CTS medium with 7 mL/L methyl oleate and 4 mL/L trace elements in batch process | 2.1 ± 0.2 | 3.3 ± 0.7 |
| Intermittent fed-batch process | 6.3 ± 0.6 | 9.8 ± 2.0 |
| Continuous fed-batch process | 7.3 ± 0.7 | 23 ± 4.6 |

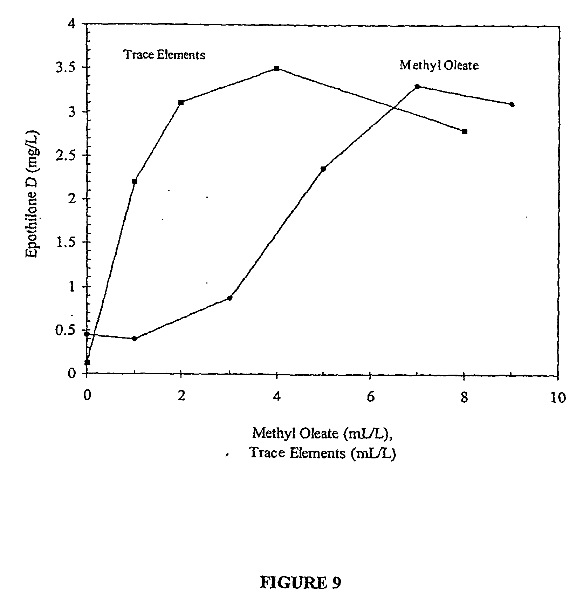

Further titer improvements were not observed at higher methyl oleate concentrations, as shown in Figure 9.

In addition to demonstrating the significance of methyl oleate on cell growth and production, the above graph also emphasizes the importance of trace elements in the production medium. In anticipation that the nutrients supplied by casitone may not be sufficient for vigorous cell growth, an exogenous addition of trace metals was added in conjunction with the methyl oleate. Surprisingly, this supplement was found to be essential for both growth and production enhancement. In its absence, the maximum biomass concentration and epothilone D titer were only 1.2 g/L and 0.12 mg/L, respectively. This low titer was comparable to that obtained with the basal medium.

### Fed-Batch Development

In the presence of optimal concentrations of methyl oleate and trace elements in a batch fermentation process, exponential growth of the M. *xanthus* strain occurred during the first two days after inoculation. Production of epothilone D began at the onset of the stationary phase and ceased when cell lysis occurred with the depletion of methyl oleate on day 5, as shown in Figures 10A and 10B. The time courses for methyl oleate consumption and ammonia generation are also shown. The concentration of ammonia in the production medium was < 4 mM throughout the course of the fermentation.

To extend the production period of the cells in the flask cultures, casitone and methyl oleate were added to the medium once a day at a rate of 2 g/L/day and 3 mL/L/day, respectively. The substrate feeds were initiated 48 h after inoculation, and the cells grew exponentially for three days thereafter. As shown in Figure 11A, the biomass concentration began to plateau on day 5 and reached a maximum at 6.3 g/L on day 10. Again, epothilone D production coincided with the stationary growth phase, and a final yield of 9.8 mg/L was attained at the end of day 12. As shown in the Figure 11B, both the consumption of methyl oleate and the generation of ammonia increased at constant rates over the course of the fermentation.

Methyl oleate was depleted at the same rate it was fed to the bioreactor, and ammonia accumulated at a rate of 3.2 mM/day. Lower feed rates of casitone or methyl oleate greatly reduced the epothilone D titer, while higher feed rates led to the premature lysis of the cells before significant production was achieved.

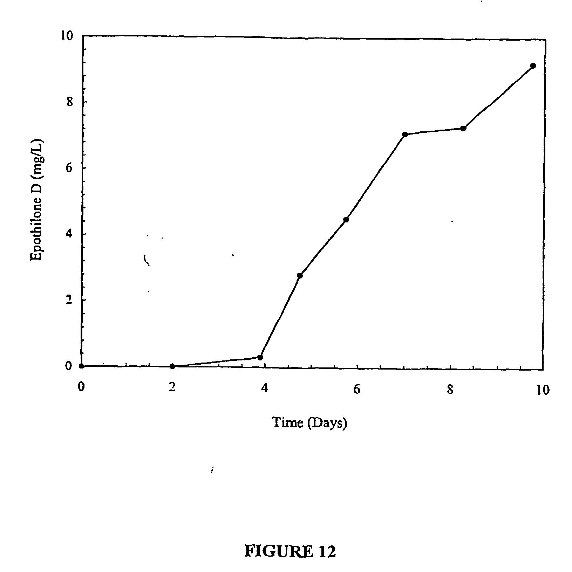

To test the effectiveness of the fed-batch process on a larger scale, casitone and methyl oleate were added intermittently at 24-h intervals to a 5-L fermentation in a bioreactor. As shown in Figure 12, the resulting production curve closely resembled that for the flask cultures. The substrate feeds were initiated 24 h after inoculation, and the production of epothilone began on day 4. A peak epothilone D titer of 9.2 mg/L was obtained ten days after inoculation.

To assess the impact of a more refined feeding strategy on growth and production, the dual feeds were delivered continuously to the bioreactor. As illustrated in Figure 13A, the implementation of the continuous feeds did not affect the growth of the cells, but it increased their productivity by nearly three-fold. A final epothilone D titer of 27 mg/L was achieved 10 days after inoculation. As shown in Figure 13B, methyl oleate was consumed at the same rate it was added to the production medium, and ammonia was released at a steady rate of 6.4 mM/day over the course of the fermentation.

### DISCUSSION

Although the chemical synthesis of epothilone D has recently been achieved (Harris *et al*., 1999; Meng *et al*., 1998; Sinha *et al*., 1998), the complex 20-step process is not an economically viable method for the large-scale production of the compound. While the initial production yield for epothilone D in *Myxococcus xanthus* strain was 0.16 mg/L, the improved fermentation processes described herein substantially increased the production level to 23 mg/L.

One of the major barriers to attaining a higher epothilone D titer was rapid degradation of the product in the fermentation broth. This problem was alleviated with the incorporation of an adsorber resin to the culture medium. The addition of XAD-16 did not affect the growth of the cells but minimized the loss of the excreted product and increased its recovery by three-fold.

Another obstacle to enhancing the production yield is the limited improvement in titer with the use of casitone as the primary carbon and nitrogen source. Although growth of the *M. xanthus* strain was stimulated with increasing casitone concentrations, a concentration that exceeded 5 g/L resulted in a dramatic decrease in titer. This inhibitory effect in secondary metabolite production at high concentrations of peptone or casitone has previously been demonstrated in several other myxobacterial fermentations and has been attributed to the accumulation of ammonia in the culture medium.

Because M. *xanthus* is incapable of metabolizing polysaccharides and sugars (Reichenbach and Dworkin, 1991), its ability to utilize oils as a carbon source was examined. Oils are attractive carbon substrates, because the oxidation of fatty acids not only can serve as a source of energy for the cells, but the formation of acetyl-CoA as a degradation product can also provide precursors for epothilone biosynthesis. The addition of oils to the fermentation of *Saccharopolyspora erythraea*, *Streptomyces fradiae,* and *Streptomyces hygroscopicus* has been shown to enhance the production of polyketide molecules, such as erythromycin, tylosin, and the immunoregulant L-683590, respectively (Mirjalili *et al*., 1999; Choi *et al*., 1998; Junker *et al*., 1998).

From a screen of different oils, methyl oleate was identified as the leading candidate in promoting cell growth and epothilone D production. These improvements, however, were observed only with the simultaneous addition of trace metals to the production medium. The sole addition of methyl oleate at 7 mL/L increased the maximum cell density from 0.4 g DCW/L to 1.2 g DCW/L, but the production remained at the baseline level. With the exogenous supplement of 4 mL/L of trace elements, the peak biomass concentration increased to 2.1 g DCW/L, and the epothilone D titer was boosted from 0.45 mg/L to 3.3 mg/L. These findings indicate that nutritional components deficient in casitone may be important for the growth of M. *xanthus* and the formation of epothilones.

With the establishment of optimal concentrations of methyl oleate and trace elements for a batch process, efforts were made to develop a feeding strategy to maintain vigorous cell growth and to prolong the production period. Intermittent and continuous feeds of casitone and methyl oleate at constant rates were evaluated, and both methods resulted in similar improvements in the growth profiles. With optimal feeds of the two substrates, maximum cell densities of about 6.8 g DCW/L were obtained. In both cases, methyl oleate was depleted as it was added to the fermentation medium.

In contrast to cell growth and methyl oleate consumption, epothilone production and ammonia generation were greatly influenced by the choice of the feeding strategy. In the continuous fed-batch culture, a peak epothilone D titer of 23 mg/L was obtained. This was nearly 2.5 times the titer obtained for the intermittent fed-batch culture. With the continuous feeds, the rate at which ammonia was released by the cells was also twice as high, suggesting a higher rate of casitone consumption. Together, these results indicate that the lower titers associated with the intermittent fed-batch process may have been caused by catabolite repression and not ammonia accumulation. Moreover, they suggest that the productivity of the M. *xanthus* strain is sensitive to the amount of substrates present in the culture medium and may be maximal under substrate-limiting conditions. This is also consistent with the observation that increasing casitone concentration in the production medium results in higher cell densities but lower titers.

Compared to the batch cultures with the basal medium, the continuous fed-batch cultures yielded a 17-fold increase in cell density and a 140-fold increase in titer. This process has been scaled from 5-L to 1000-L and shown to perform equivalently. The results shown for producing epothilone D on a manufacturing scale demonstrate that M. *xanthus* can be used as a host for the production of other biologically active molecules from myxobacteria.

### REFERENCES

Bollag et *al.* 1995. Epothilones, a new class of microtubule-stabilizing agents with a taxol-like mechanism of action. Cancer Res 55:2325-2333.

Choi *et al.* 1998. Effects of rapeseed oil on activity of methylmalonyl-CoA carboxyltransferase in culture of *Streptomyces fradine*. Biosci Biotechnol Biochem 62:902-906.

Chou *et al*. 1998. Desoxyepothilone B: An efficacious microtubule-targeted antitumor agent with a promising in vivo profile relative to epothilone B. Proc Natl Acad Sci USA 95:9642-9647.

Gerth *et al.* 1996. Epothilons A and B: Antifungal and cytotoxic compounds from *Sorangium cellulosum* (myxobacteria) - production, physico-chemical, and biological properties. J Antibiot (Tokyo) 49:560-563.

Gerth *et al.* 2000. Studies on the biosynthesis of epothilones: the biosynthetic origin of the carbon skeleton. J Antibiot (Tokyo) 53:1373-1377.

Gerth *et al*. 2001. Studies on the biosynthesis of epothilones: The PKS and epothilone C/D monooxygenase. J Antibiot (Tokyo) 54:144-148.

Harris *et al*. 1999. New chemical synthesis of the promising cancer chemotherapeutic agent 12,13-desoxyepothilone B: Discovery of a surprising long-range effect on the diastereoselectivity of an aldol condensation. J Am Chem Soc 12:7050-7062.

Hecht *et al*. 1990. Hollow fiber supported gas membrane for *in situ* removal of ammonium during an antibiotic fermentation. Biotechnol Bioeng 35:1042-1050.

Junker *et al*. 1998. Use of soybean oil and ammonium sulfate additions to optimize secondary metabolite production. Biotechnol Bioeng 60:580-588.

Kowalski *et al*. 1997. Activities of the microtubule-stabilizing agents epothilones A and B with purified tubulin and in cells resistant to paclitaxel. J Biol Chem 272:2534-41.

Meng *et al*. 1997. Remote effects in macrolide formation through ring-forming olefin metathesis: An application to the synthesis of fully active epothilone congeners. J Am Chem Soc 119:2733-2734.

Mirjalili *et al*. 1999. The effect of rapeseed oil uptake on the production of erythromycin and triketide lactone by *Saccharopolyspora erythraea*. Biotechnol Prog 15:911-918.

Molnár et *al*. 2000. The biosynthetic gene cluster for the microtubule-stabilizing agents epothilones A and B from *Sorangium cellulosum* So ce90. Chem Biol 7:97-109.

Reichenbach *et al*. The Prokaryotes II Eds. New York: Springer-Verlag. p 3417-3487.

Reichenbach *et al*. 1993. Production of Bioactive Secondary Metabolites. In: Dworkin M, Kaiser D, editor. Myxobacteria II: Washington, DC: American Society for Microbiology, p 347-397.

Sinha *et al*. 1998. The antibody catalysis route to the total synthesis of epothilones. Proc Natl Acad Sci USA 95:14603-14608.

Su *et al*. 1997. Structure-activity relationships of the epothilones and the first *in vivo* comparison with paclitaxel. Angew Chem Int Ed Engl 36:2093-2096.

Tang *et al*. 2000. Cloning and heterologous expression of the epothilone gene cluster. Science 287:640-642.

### B. Production of Epothilone B

### Flasks

A 1 mL vial of the K111-32-25 strain is thawed and the contents transferred into 3 mL of CYE seed media in a glass tube. This culture is incubated for 72±12 hours at 30°C, followed by the subculturing of 3 mL of this tube culture into 50 mL of CYE media within a 250 mL baffled Erlenmeyer flask. This CYE flask is incubated for 24±8 hours at 30°C, and 2.5 mL of this seed (5% v/v) used to inoculate the epothilone production flasks (50 mL of CTS-TA media in a 250 mL baffled Erlenmeyer flask). These flasks are then incubated at 30°C for 48±12 hours, with a media pH at the beginning of 7.4.

### Fermentors

A similar inoculum expansion of K111-32-25 as described above is used, with the additional step that 25 mL of the 50 mL CYE seed is subcultured into 500 mL of CYE. This secondary seed is used to inoculate a 10 L fermentor containing 9.5 L of CTS-TA, and 1 g/L of sodium pyruvate. The process parameter setpoints for this fermentation are: pH - 7.4; agitation - 400 rpm; sparge rate - 0.15 vvm. These parameters were sufficient to maintain the DO at greater than 80% of saturation The pH control is provided by addition of 2.5 N sulfuric acid and sodium hydroxide to the cultures. Peak epothilone titers are achieved at 48±8 hours.

### C. Production of Epothilone D

### Flasks

A 1 mL vial of the K111-40-1 strain (described in Example 4) is thawed and the contents transferred into 3 mL of CYE seed media in a glass tube. This culture is incubated for 72±12 hours at 30°C, followed by the subculturing of 3 mL of this tube culture into 50 mL of CYE media within a 250 mL baffled Erlenmeyer flask. This CYE flask is incubated for 24±8 hours at 30°C, and 2.5 mL of this seed (5% v/v) used to inoculate the epothilone production flasks (50 mL of 1x wheat gluten media in a 250 mL baffled Erlenmeyer flask). These flasks are then incubated at 30°C for 48±12 hours, with a media pH at the beginning of 7.4.

### Fermentors

A similar inoculum expansion of K111-40-1 as described above is used, with the additional step that 25 mL of the 50 mL CYE seed is subcultured into 500 mL of CYE. 250 mL of this secondary seed is used to inoculate a 5 L fermentor containing 4.5 L of CTS-TA, with a 1 g/L daily feed of sodium pyruvate. The process parameter setpoints for this fermentation are: pH - 7.4; agitation - 400 rpm; sparge rate - 0.15 vvm. These parameters were sufficient to maintain the DO at greater than 80% of saturation. The pH control is provided by addition of 2.5 N sulfuric acid and sodium hydroxide to the cultures. Peak epothilone titers are achieved at 36±8 hours. The peak epothilone C titer is 0.5 mg/L, and the peak epothilone D titer is 1.6 mg/L.

Table 5 is a summary of the media that were used and their respective components.

**TABLE 5**

| **CYE Seed Media** | **Component** | **Concentration** |
|---|---|---|
| | Casitone (Difco) | 10 g/L |
| | Yeast Extract (Difco) | 5 g/L |
| | MgSO₄-7H₂0 (EM Science) | 1 g/L |
| | HEPES buffer | 50 mM |

| **CTS-TA Production Media** | **Component** | **Concentration** |
|---|---|---|
| | Casitone (Difco) | 5 g/L |
| | MgSO₄·7H₂0 (EM Science) | 2 g/L |
| | L-alanine, L-serine, glycine | 1 mg/L |
| | HEPES buffer | 50 mM |

| **1x Wheat Gluten Production Media** | **Component** | **Concentration** |
|---|---|---|
| | Wheat Gluten (Sigma) | 5 g/L |
| | MgSO₄·7H₂0 (EM Science) | 2 g/L |
| | HEPES buffer | 50 mM |

The CYE seed media and the CTS-TA production media are sterizlied by autoSterilized autoclaving for 30 minutes at 121 °C. The wheat gluten production media is sterilized by autoclaving for 45 minutes at at 121°C.

### D. Production of Epothilone C and D from Myxococcus xanthus

In one aspect, an improved fermentation process for *Myxococcus* strains, including but not limited to *M. xanthus* K111-40-1 may be provided, in which the fermentation media provides carbon sources that can be utilized without generation of ammonia. The carbon source may be an oil, such as methyl oleate or a similar oil. In shake flask tests with a variety of feed ratios, these methods resulted in the production of epothilones C and D, predominantly epothilone D, at levels ranging from 15 to 25 mg/L, as described below.

### Seed Culture

A frozen 1 mL vial of M. *xanthus* K111-40-1 cells that had been grown in 50 mL of CYE medium with 2 mL/L methyl oleate was used to inoculate 3 mL of fresh CYE medium with 1 mL/L methyl oleate in a sterile glass tube. The tube was incubated at 30°C in a 250 RPM shaker for 24 hrs. The inoculum in the glass tube was then transferred into a 250 mL unbaffled flask that contained 50 mL of fresh CYE medium with 2 mL/L methyl oleate. The flask was incubated at 30°C in a 250 RPM shaker for 48 hrs.

### Production Flask

1 g of Amberlite XAD-16 was sterilized in a 250-mL unbaffled flask by autoclaving at 121°C for 30 min. 50 mL of sterile production media were then added to the flask. The flask was inoculated with 5% (v/v) of the seed culture and was placed in an incubator shaker operating at 250 RPM and 30°C. A 3 mL/L/day feed of sterile methyl oleate was initiated two days after the time of inoculation, and a 2 g/L/day feed of casitone was initiated one day after the time of inoculation.

### Product Extraction

After 14 days, the XAD resin in the production flask was transferred into a 50 mL centrifuge tube. Excess medium in the tube was decanted without removing any of the resin. The XAD resin was then washed with 25 mL of water and allowed to settle. The water in the tube was decanted without removing any of the resin, and 20 mL of methanol were added to the tube. The centrifuge tube was placed on a shaker at 175 RPM for 20-30 min. to extract the epothilone products from the resin. The methanol extract was transferred to a new centrifuge tube for storage and LC/MS analysis.

Table 6 is a summary of the media that were used and their respective components.

**TABLE 6**

| **CYE Media** | **Component** | **Concentration** |
|---|---|---|
| | Casitone (Difco) | 10,g/L |
| | Yeast Extract (Difco) | 5 g/L |
| | MgSO₄-7H₂O (EM Science) | 1 g/L |

| **Production Media** | **Component** | **Concentration** |
|---|---|---|
| | Casitone (Difco) | 5 g/L |
| | MgSO₄-7H₂0 (EM Science) | 2 g/L |
| Added to Production Media after autoclaving | | |
| | 1000x Trace Element Solution | 4 mL/L |
| | Methyl Oleate | 2 mL/L |

The CYE media and the production media are sterizlied by autoSterilized autoclaving for 30 minutes at 121°C. The trace element solution is filter-sterilized; the methyl oleate is autoclaved separately.

Trace element solution is made by combining all of the components in Table 7, adding 10 mL/L concentrated H₂SO₄ to the solution and brining the final volume to 1 L.

**TABLE 7**

| **1000x Trace Element Solution** | **Component** | **Concentration** |
|---|---|---|
| | FeCl₃ | 8.6 g/L |
| | ZnCl₂ | 2.0 g/L |
| | MnCl₂·4H₂O | 1.0 g/L |
| | CuCl₂·2H₂O | 0.43 g/L |
| | H₃BO₃ | 0.31 g/L |
| | CaCl₂·6H₂O | 0.24 g/L |
| | Na₂MoO₄·2H₂O | 0.24 g/L |

The resulting solution is filtered sterilized.

### E. Fermentation, Production, and Purification ofEpothilones from Myxococcus xanthus

### Description of M. xanthus strains

Strain K111-25-1 is the epothilone B producing strain, which also produces epothilone A. Strain K111-40-1 is the epothilone D producing strain, which also produces epothilone C.

### Maintainance of M. xanthus on plates

The *M. xanthus* strains are maintained on CYE agar plates (see Table 8 for plate composition). Colonies appear approximately 3 days after streaking out on the plates. Plates are incubated at 32°C for the desired level of growth and then stored at room temperature for up to 3 weeks (storage at 4°C on plates can kill the cells).

**TABLE 8**

| **CYE Agar Plates*** | **Component** | **Concentration** |
|---|---|---|
| | Hydrolyzed casein (pancreatic digest) | 10 g/L |
| | Yeast extract | 5 g/L |
| | Agar | 15 g/L |
| | MgSO₄ | 1 g/L |
| | 1 M MOPS buffer solution (pH 7.6) | 10 mL/L |

| | | |
|---|---|---|
| *1 L agar media batches are autoclaved for 45 minutes, then poured out into petri dishes. | | |

### Oil adaptation of M. xanthus for Cell Banking

Transfer a non-oil adapted colony from a CYE plate or a frozen vial of cells into a 50 mL glass culture tube containing 3 mL of CYE seed media and 1 drop of methyl oleate from a 100 µL pipet. Allow cells to grow for 2-6 days (30°C, 175 rpm) until the culture appears dense under a microscope. Start several (5-7) tubes in parallel, as these cells do not always adapt well to the oil.

### Cell banking procedure (Master Cell Bank)

Start an oil-adapted tube culture as described above. When the tube culture is sufficiently dense (OD = 5 +/-1), transfer the entire contents of the tube into a sterile 250 mL shake flask containing 50 mL of CYE-MOM seed media (see table below for media composition). After 48 ± 12 hours of growth in a shaker incubator (30°C, 175 rpm), transfer 5 mL of this seed culture into 100 mL of CYE-MOM in a 500 mL shake flask. Allow this culture to grow for 1 day in a shaker incubator (30°C, 175 rpm). Check culture microscopically for appropriate growth and lack of contamination.

Combine 80 mL of this seed culture and 24 mL of sterile 90% glycerol in a sterile 250 mL shake flask. Swirl to thoroughly mix, and aliquot 1 mL of this mixture into 100 sterile, prelabeled cryovials. Slow freeze vials by placing them in a -80°C freezer.

### Cell banking procedure (Working Cell Bank)

Start a tube culture by thawing one of the master cell bank vials produced as described above at room temperature, then depositing its entire contents into a glass tube containing 3 mL of CYE-MOM seed media. When this tube culture is sufficiently dense (OD = 5 +/-1), transfer the entire contents of the tube into a sterile 250 mL shake flask containing 50 mL of CYE-MOM seed media. After 48 ± 12 hours of growth (at 30°C, 175 rpm), transfer 5 mL of this seed culture into 100 mL of CYE-MOM in a 500 mL shake flask. Allow this to grow for 1 day (30°C, 175 rpm). Check microscopically for growth and contamination.

Combine 80 mL of this seed culture and 24 mL of sterile 90% glycerol in a sterile 250 mL shake flask. Swirl to thoroughly mix, and aliquot 1 mL of this mixture into 100 sterile, prelabeled cryovials. Slow freeze vials by placing them in a -80°C freezer.

### Composition of Seed Media

The same seed media as described by Table 9 is used for cell banking and the expansion of the cell bank vials up to any required volume.

**TABLE 9**

| **CYE-MOM Seed Medium*** | **Component** | **Concentration** |
|---|---|---|
| | Hydrolyzed casein (pancreatic digest) - Difco | 10 g/L |
| | Yeast extract - Difco | 5 g/L |
| | MgSO₄·7H₂O - EM Science | 1 g/L |
| | Methyl Oleate - Cognis | 2 mL/L |

| | | |
|---|---|---|
| ***Note:** the methyl oleate is added after the other ingredients, as it forms an emulsion in the casitone and does not completely mix with the other components. | | |

### Inocula Scaleup for Shake Flask, 5L, and 1000L Fermentations

Thaw a frozen working cell bank vial of the methyl oleate adapted cells. Transfer the entire contents of the vial into a 50 mL glass culture tube, containing 3 mL of the CYE-MOM seed media. Place tube in a shaker (30°C, 175 rpm), and grow for 48 ± 24 hours. Transfer the entire contents of the culture tube into a 250 mL shake flask containing 50 mL of CYE-MOM seed media. Place flask in a shaker (30°C, 175 rpm) and grow for 48 ± 24 hours. For use in shake flask experiments, expand this seed by subculturing 10 mL of this culture into 40 mL of fresh CYE-MOM in 5 new seed flasks. Incubate seed flasks in shaker (30°C, 175 rpm) for 24 ± 12 hours for use as an inoculum for flask volume (30 -100 mL) production cultures. Inoculate production flasks at 4.5% of the combined (seed and production media) initial volume.

To prepare seeds for small scale (5 -10 L) fermentations, subculture the entire contents of one of these 50 mL seed flasks into a sterile 2.8 L fernbach flask containing 500 mL of CYE-MOM. Incubate this fernbach flask in a shaker (30°C, 175 rpm) for 48 ± 24 hours for use as the fermentor inoculum. Inoculate the production fermentation at about 5% of the combined initial volume.

Further seed expansion is required for large scale (1000 L) fermentations. Here, 1 L of the fernbach flask seed is used to inoculate (5% by volume) a 10 L seed fermentor containing 9 L of CYE-MOM. The fermentor pH is controlled at 7.4 by addition of 2.5 N potassium hydroxide and 25 N sulfuric acid. The temperature is set at 30°C. The dissolved oxygen is maintained at or above 50% of saturation by cascading of the stir rate between 400 - 700 rpm. The initial agitation rate is set at 400 rpm, and the sparging rate was maintained at 0.1 v/v/m. After 24 ± 12 hours of growth in the 10L fermentor, the entire culture is transferred into a 150 L fermentor containing 90 L CYE-MOM. The pH is once again controlled at 7.4 with 2.5 N potassium hydroxide and 2.5 N sulfuric acid. The temperature is set at 30°C. The dissolved oxygen is maintained at or above 50% of saturation by cascading of the stir rate between 400 - 700 rpm. The initial agitation rate is set at 400 rpm, and the sparging rate is maintained at 0.1 v/v/m.

### Example 4

### Construction of a Myxococcus Strain with a Non-functional epoK Gene

Strain K111-40-1 was constructed from strain K111-32.25 by insertional inactivation of the *epoK* gene. To construct this *epoK* mutant, a kanamycin resistance cassette was inserted into the *epoK* gene. This was done by isolating the 4879 bp fragment from pKOS35-79.85, which contains *epoK*, and ligating it into the *Not*I site of pBluescriptSKII+. This plasmid, pKOS35-83.5, was partially cleaved with *Sca*I, and the 7.4 kb fragment was ligated with the 1.5 kb *Eco*RI*-Bam*HI fragment containing the kanamycin resistance gene from pBJ180-2, which had the DNA ends made blunt with the Klenow frangment of DNA polymerase I, to yield plasmid pKOS90-55. Finally, the ~400 bp RP4 oriT fragment from pBJ183 was ligated into the *Xba*I and *Eco*RI sites to create pKOS90-63. This plasmid was linearized with *Dra*I and electroporated into the *Myxococcus xanthus* strain K111-32.25, and transformants selected to provide *M. xanthus* strain K111-40.1. Strain K111-40.1 was deposited in compliance with the Budapest Treaty with the American Type Culture Collection, Manassas, VA 20110-2209, USA on Nov. 21, 2000, and is available under accession No. PTA-2712.

To create a markerless *epoK* mutation, pKOS35-83.5 was cleaved with *Sca*I and the 2.9 kb and 4.3 kb fragments were ligated together. This plasmid, pKOS90-101, has an in-frame deletion in *epoK*. Next, the 3 kb *Bam*HI and *Nde*I fragment from KG2, which had the DNA ends made blunt with the Klenow fragment of polymerase I and contains the kanamycin resistance and *galK* genes, was ligated into the *Dra*I site of pKOS90-101 to create pKOS90-105. This plasmid was electroporated into K111-32.25 and kanamycin resistant electroporants were selected. To replace the wild type copy of *epoK* with the deletion, the second recombination event was selected by growth on galactose plates. These galactose resistant colonies are tested for production of epothilone C and D, and a producing strain was designated K111-72.4.4 and deposited in compliance with the Budapest Treaty with the American Type Culture Collection, Manassas, VA 20110-2209, USA on Nov. 21, 2000, and is available under accession No. PTA-2713.

### Example 5

### Addition of matBC

The *matBC* genes encode a malonyl-CoA synthetase and a dicarboxylate carrier protein, respectively. See An and Kim,1998, *Eur. J. Biochem.* 257: 395-402. These two proteins are responsible for the conversion of exogenous malonate to malonyl-CoA inside the cell. The products of the two genes can transport dicarboxylic acids and convert them to CoA derivatives (see PCT patent application No. US00/28573). These two genes can be inserted into the chromosome of *Myxococcus xanthus* to increase the cellular concentrations of malonyl-CoA and methymalonyl-CoA to increase polyketide production. This is accomplished by cleaving pMATOP-2 with *Bgl*II and *Spe*I and ligating it into the *Bgl*II and *Spe*I sites of pKOS35-821, which contains the tetracycline resistance conferring gene, the Mx8 att site and the *M. xanthuts pilA* promoter to drive expression of *matBC*. This plasmid can be electroporated into M. *xanthus*. Because the *pilA* promoter is highly transcribed, it may be necessary to insert a weaker promoter in the event that too much MatB and MatC affect cell growth. Alternative promoters include the promoter of the kanamycin resistance conferring gene.

### Example 6

### Production of Epothilone Analogs

### A. Production of 13-keto-epothilone Analogs

Inactivation of the KR domain in extender moduler 4 of the epothilone PKS results in a hybrid PKS of the invention useful in the production of 13-keto epothilones. The extender module 4 KR domain was modified by replacing the wild-type gene with various deleted versions as described below. First, fragments were amplified using plasmid pKOS39-118B (a subclone of the *epoD* gene from cosmid pKOS35-70.4) as a template. The oligonucleotide primers for forming the left side of the deletion were TL3 and TL4, shown below: TL3: 5'-ATGAATTCATGATGGCCCGAGCAGCG; and TL4: 5'-ATCTGCAGCCAGTACCGCTGCCGCTGCCA. The oligonucleotide primers for forming the right side of the deletion were TL5 and TL6, shown below: TL5: 5'-GCTCTAGAACCCGGAACTGGCGTGGCCTGT; and TL6: 5-GCAGATCTACCGCGTGAGGACACGGCCTT. The PCR fragments were cloned into vector Litmus 39 and sequenced to verify that the desired fragments were obtained. Then, the clone containing the TL3/TL4 fragment was digested with restriction enzymes PstI and *Bam*HI, and the ~4.6 kb fragment was isolated. The 2.0 kb PCR fragment obtained using primers TI5/TL6 was treated with restriction enzymes *Bgl*II and XbaI and then ligated to either (i) the "short" KR linkers TL23 and TL24 (that are annealed together to form a double-stranded linker with single-stranded overhangs) to yield pKOS122-29; or (ii) the "long" (epoDH3*) linker, obtained by PCR using primers TL33+TL34 and then treatment with restriction enzymes *Nsi*I and *SpeI,* to yield plasmid pKOS122-30. The sequences of these oligonucleotide linkers and primers are shown below: TL23: 5'-GGCGCCGGCCAAGAGCGCCGCGCCGGTCGGCGGGCCAGCCGGGGACGGGT; 
TL24: 5'-CTAGACCCGTCCCCGGCTGGCCCGCCGACCGGCGCGGCGCTCTTGGCCG-
GCGCCTGCA;
 TL33: 5'-GGATGCATGCGCCGGCCGAAGGGCTCGGA; and

TL34: 5'-TCACTAGTCAGCGACACCGGCGCTGCGTTT.

The plasmids containing the desired substitution were confirmed by sequencing and then digested with restriction enzyme *Dra*I. Then, the large fragment of each clone was ligated with the kanamycin resistance and *galK* gene (KG or *kan-gal*) cassette to provide the delivery plasmids. The delivery plasmids were transformed into epothilone B producer *Myxococcus* xanthus K111-32.25 by electroporation. The transformants were screened and kanamycin-sensitive, galactose-resistant survivors were selected to identify clones that had eliminated the KG genes. Confirmation of the KG elimination and the desired gene replacement for the recombinant strains was performed by PCR. The recombinant strains were fermented in flasks with 50 mL of CTS medium and 2% XAD-16 for 5 days, and epothilone analogs were eluted from XAD with 10 mL of methanol. Structure determination was based on the LC/MS spectrum and NMR. One such strain, designated K122-56, was deposited with the American Type Culture Collection, Manassa, VA 20110-2209, USA, on Nov. 21, 2000, under the terms of the Budapest Treaty and is available under accession No. PTA-2714. The K122-56 strain (derived from plasmid pKOS122-29) produces 13-keto-11,12-dehydro-epothilone D as a major product whose structure is shown below

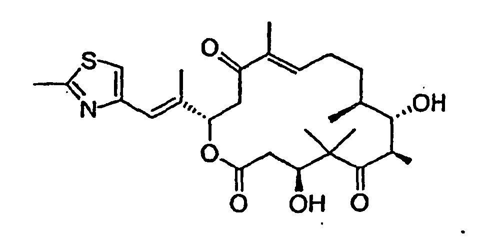

The K122-56 strain also produces 13-keto-epothilones C and D as minor products whose respective structures are shown below

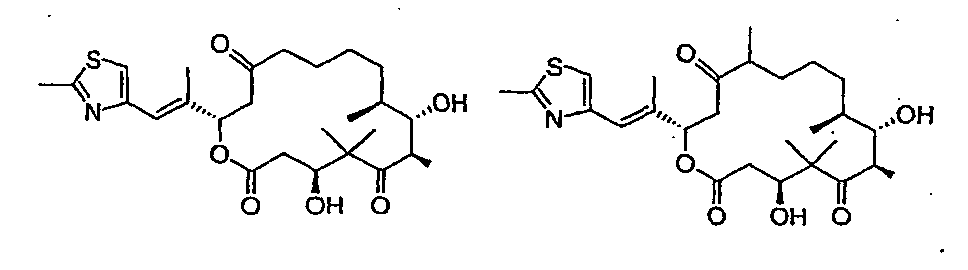

Similar results were obtained from strain K122-30, derived from plasmid pKOS122-30. These compounds and the strains and PKS enzymes that produce them are novel compounds, strains, and PKS enzymes .

Other strains that produce the 13-keto-11,12-dehydroepothilones include those in which the KR domain is rendered inactive by one or more point mutations. For example, mutating the constitutive tyrosine residue in the KR domain to a phenylalanine results in about a 10% decrease in KR activity and results in some production of 13-keto-epothilones. Additional mutations in the KR domain can eliminate more or all of the KR activity but can also lead to decreased epothilone production.

### B. Production of 13-hydroxy-epothilone Analogs

Replacement of the extender module 5 KR, DH, and ER domains of the epothilone PKS with a heterologous KR domain, such as the KR domain from extender module 2 of the rapamycin PKS or extender module 3 of the FK520 PKS, results in a hybrid PKS useful in the production of 13-hydroxy epothilones. This construction is carried out in a manner similar to that described in part A of this example. The oligonucleotide primers for amplifying the desired portions of the *epoD* gene, using plasmid pKOS39-118B as a template, were: TL7: 5'-GCGCTCGAGAGCGCGGGTATCGCT; TL8: 5'-GAGATGCATCCAATGGCGCTCACGCT; TL9: 5'-GCTCTAGAGCCGCGCGCCTTGGGGCGCT; and

TL10: 5-GCAGATCTTGGGGCGCTGCCTGTGGAA.

The PCR fragment generated from primers TL7/TL8 was cloned into vector LITMUS 28, and the resulting clone was digested with restriction enzymes *Nsi*I and *Bgl*II, and the 5.1 kb fragment was isolated and ligated with the 2.2 kb PCR fragment generated from TL9/TL10 treated with restriction enzymes *Bgl*II and *Xba*I and ligated to the KR cassettes. The KR cassette from the FK520 PKS was generated by PCR using primers TL31 and TL32 and then digestion with restriction enzymes *Xba*I and *Pst*I. These primers are shown below: TL31: 5'-GGCTGCAGACCCAGACCGCGGCGACGC; and

TL32: 5'-GCTCTAGAGGTGGCGCCGGCCGCCCGGCG.

The remainder of the strain construction proceeded analogously to that described in part A of this Example, except that *Myxococcus* xanthus K111-72.4.4 was used as the recipient. The strain in which the KR domain of extender module 3 of the FK520 PKS replaced the KR, DH, and ER domains of extender module 5 of the epothilone PKS was designated K122-148 and deposited with the American Type Culture Collection, Manassas, VA 20110-2209, USA, on Nov. 21, 2000, under the terms of the Budapest Treaty and is available under accession No. PTA-2711. Strain K122-148 produces 13-hydroxy-10,11-dehydro epothilone D as a major product and the C derivative as a minor product whose structures are shown below

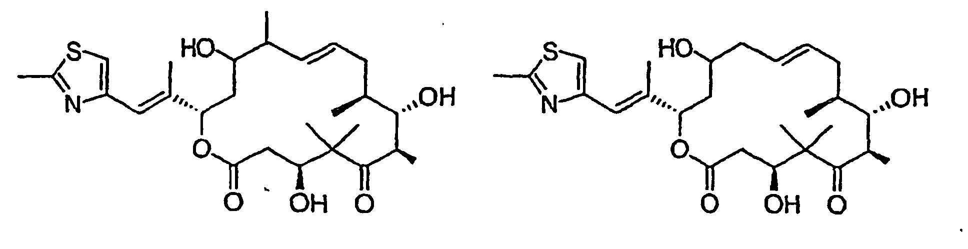

A similar strain, designated K122-52, in which the KR domain of extender module 2 of the rapamycin PKS was used for the replacement, produced the same compounds. These compounds and the strains and PKS enzymes that produce them are novel compounds, strains, and PKS enzymes of the invention.

### I. Production of Oxazole-Containing Epothilones by Fermentation

A method for obtaining the oxazole containing epothilones (in which the thiazole moiety of the corresponding epothilone is replaced by an oxazole) may be provided by fermenting an epothilone producing strain, such as a *Sorangium cellulosum* strain or a *Myxococcus* strain described herein, in media supplemented with L-serine.

To illustrate this aspect, a cultures of Myxococcus xanthus strain K111-40.1 or K111-72.4.4 is fermented in accordance with the methods of Example 3, except that L-serine is present at 11x, 51x, 101x, and 201x the basal serine concentration in the batch media (2.3 mM). The batch media-containing 50 mL cultures thus contain: 20 g/L XAD-16; 5 g/L casitone; 2 g/L MgSO4; 7 mL/L methyl oleate; and 4 mL/L trace metals solution, and an appropriate concentration of a filter-sterilized 1.25 M solution of L-serine is added. The batch titers observed in basal media were: Epo C: 0.4 mg/L, Epo D: 2 mg/L, Epo H1 (the C analog of the oxazole): Not detectable, and Epo H2 (the D analog of the oxazole): 0.02 mg/L. Increasing the serine concentration decreased the epoC and epoD concentrations (almost to undetectable levels at 51x supplementation). Thus, the batch titers in 51x supplementation of L-serine in basal media were: Epo C: 0.03 mg/L, Epo D: 0.05 mg/L, Epo H1: 0.12 mg/L, and Epo H2: 0.13. mg/L. A fed-batch protocol could increase the observed titers by about 10 fold.

### J. Construction of Epothilone Analogs

Epothilones and epothilone derivatives may be produced by recombinant epothilone PKS enzymes in which (i) the specificity of the extender module 1 NRPS has been changed from cysteine to another amino acid; (ii) the loading domain has been changed to an NRPS or CoA ligase; or (iii) both (i) and (ii). This example describes how such recombinant epothilone PKS enzymes are constructed; references cited in this example are listed at the end of this example and are referred to in the text by a citation number.

Epothilones contain the amino acid cysteine that has been cyclized and oxidized to form the thiazole. Two other amino acids, serine and threonine, can undergo similar cyclization and oxidation to yield an oxazole and methyloxazole, respectively. For example, the oxazole and methyloxazole derivatives of epothilone D are shown below

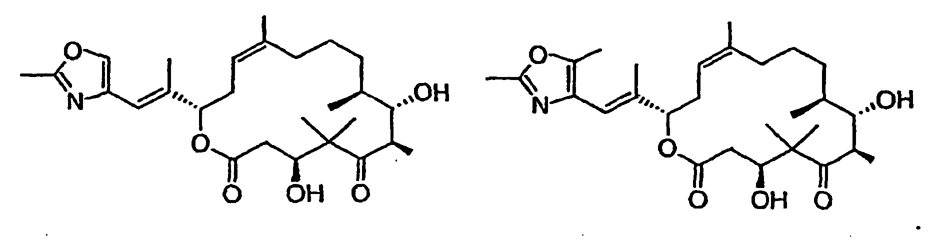

To construct analogs of epothilone with either the oxazole or methyloxazole, engineering of extender module 1, the NRPS module, can be performed. NRPS modules that extend a growing molecule are minimally composed of a domain that activates an amino acid, the adenylation domain, a PCP or peptidyl carrier protein domain, which tethers the amino acid to the NRPS, and a condensation domain, which condenses the amino acid to a carboxyl group of the growing molecule to form a peptide bond (5, 7). The recognition sequence for determining the specificity of the amino acid is found within the adenylation domain, specifically between the A4 and A5 consensus sequence (4). Analysis of the region has shown that key amino acids in this protein region can predict which amino acid will be used by the NRPS (2, 8). Experiments have been performed that exchange the complete NRPS adenylation region for that of another, which results in a hybrid NRPS that has the amino acid specificity of the new adenylation domain (6, 9). Experiments using smaller regions of the adenylation region, such as the one between the A4 and A5 consensus sequence have not been reported. In one embodiment, a hybrid PKS of the invention is constructed by replacing the region between the A4 and A5 consensus region of the adenylation domain from *epoB* with those from *vibF* and *blmVII*, which utilize threonine, and with the NRPS-4 region from *blmVI* of the bleomycin gene cluster, which utilizes serine (3).

Recent experiments suggest that the condensation domain may be able to detect if an incorrect amino acid has been attached to the PCP (1). Once an incorrect amino acid is detected the efficiency of the condensation reaction is reduced. To avoid this, in addition to swapping the adenylation domain, one can also bring along the cognate condensation domain in order to change the specificity of the adenylation domain and engineer a fully active NRPS.

Recombinant epothilone PKS enzymes may be provided that produce the 16 desmethyl derivatives of the oxazole and methyloxazole forms of epothilone. Such enzymes are constructed by changing the AT domain of extender module 2, *epoC*, from methymalonyl specific to malonyl specific. AT domains that can be used to make the constructs include those from extender module 5 and 9 of the epothilone cluster and extender modules 2 and 4 from the soraphen gene cluster.

Recombinant PKS enzymes may be provided in which the EpoA protein has been replaced by an NRPS. Novel epothilones may be produced by such enzymes. Epothilone biosynthesis begins by the loading of malonate onto the ACP of the loading module, EpoA. This malonate is subsequently decarboxylated by the KS domain and then transferred as an acetyl moiety to EpoB, the NRPS module. After the molecule has been acted on by EpoB, the resulting compound is 2-methylthiazole.

To make analogs that have an amino acid attached at the 2 position on the thiazole, deletion of *epoA* and the insertion of a NRPS module are needed. Any NRPS module can be used; however, to make the most conservative change, one can employ an NRPS module to replace *epoA* that naturally communicates with a downstream NRPS module. Moreover, because the NRPS replacing epoA is the loading module, it does not need a condensation domain. This can be done by taking an extender NRPS module and removing the condensation domain or using an NRPS that is naturally a loading module and thus lacks the condensation domain. An illustrative NRPS loading module is the one from *safB,* which utilizes alanine and is from a *M. xanthus*.

In constructing M. *xanthus* strains that contain the *safB* loading module in the place of *epoA,* one can determine the optimum boundaries for the new loading module and *epoB.* The linker region between PKS proteins is often critical for "communication" between those proteins. One can construct three different strains to examine the optimum linker. In the first, the ACP domain of EpoA is fused to the adenylation domain of loading domain of *safB*. This construct requires that the ACP of EpoA function as a PCP. Although PCP and ACP domains are functionally similar, they do not show high sequence identity and thus may be restricted on what they can recognize and bind. The second construct fuses the last several amino acids of EpoA downstream of the PCP domain of the SafB loading module, thus providing the necessary linker region for the hybrid loading module to "communicate" with EpoB. Finally, a fusion of the SafB loading module with EpoB will be constructed. Because SafB is composed of two modules, it is possible to take all of the loading module of SafB and fuse it directly to EpoB to give a fusion protein, which should optimal for communication between the SafB loading module and EpoB.

Once SafB or any another loading NRPS domain has been used to replace the 2-methyl on the thiazole of epothilone with an amino acid, then changes can be made in the new loading NRPS module so that any amino acid could be used to start the synthesis of the epothilone analogs. A comprehensive list of potential amino acids and their corresponding NRPS modules that could be used for these swaps are provide by Challis *et al.* (2).

All of the replacements can be made in K111-32.25, the M. *xanthus* strain that contains the epothilone genes, or K111-40-1, the M. *xanthus* strain that contains the epothilones genes and in which the *epoK* gene does not produce a functional product, or any other epothilone producing strain of the invention or in *Sorangium cellulosum.* In Myxococcus, the appropriate constructs can be made on plasmids and, using the *galK* and kanamycin selection, used to replace the wild type genes with the engineered ones. For example, a replacement of the NRPS in K111-40-1 with an NRPS specific for serine is expected to make the 2-methyl-oxazole derivative of epothilone D and 2-methyl-oxazole derivative of epothilone C as the major and minor products respectively. The structure of these compounds are shown below

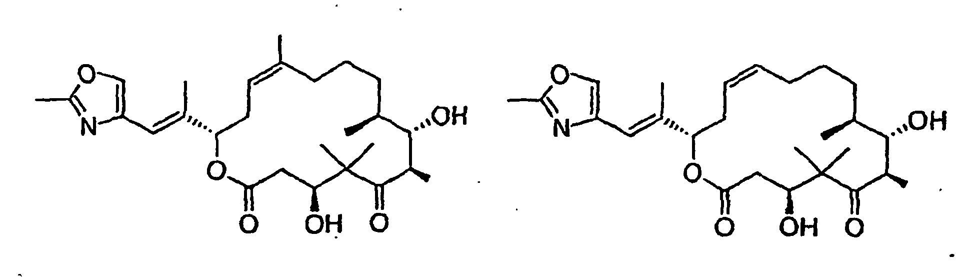

Replacement of the NRPS in K111-40-1 with an NRPS specific for threonine is expected to the the following compounds and the major and minor products respectively

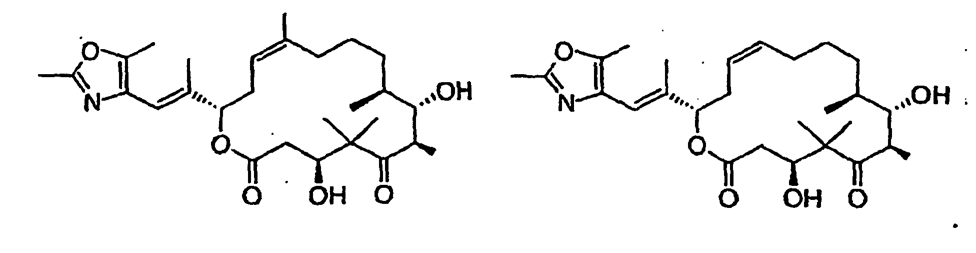

Replacement of the NRPS in K111-40-1 with an NRPS specific for glycine, alanine, glutamic acid, aspartic acid, phenylalanine, histidine, isoleucine, leucine, methionine, asparagine, glutamine, arginine, valine, and tyrosine are each expected to make the following compounds as the major and minor products - respectively 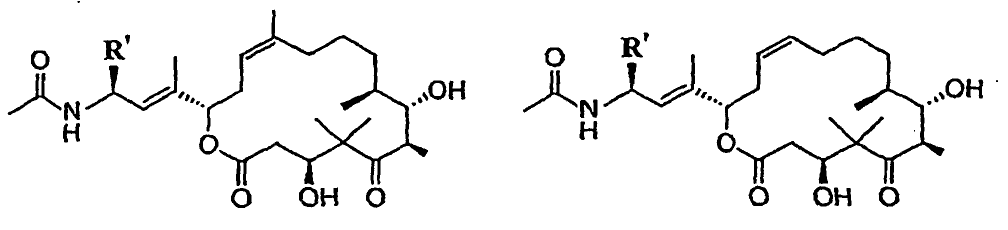 where R' corresponds to the specific side chain in the amino acid (for example, R' is H in the general amino acid formula NH₂-CHR'COOH for glycine and is methyl for alanine and so on).

Replacement of the NRPS in K111-40-1 with an NRPS specific for proline is expected to make the following compounds as the major and minor products respectively

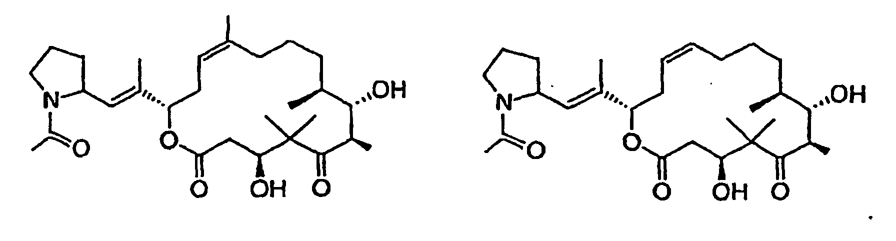

The references cited in this subsection are as follows.
1 Belshaw *et al*. 1999. Aminoacyl-CoAs as probes of condensation domain selectivity in nonribosomal peptide synthesis Science. 284:486-9.
2. Challis *et al*. 2000. Predictive, structure-based model of amino acid recognition by nonribosomal peptide synthetase adenylation domains Chem Biol. 7:211-24.
3. Du *et al*. 2000. The biosynthetic gene cluster for the antitumor drug bleomycin from Streptomyces verticillus ATCC15003 supporting functional interactions between nonribosomal peptide synthetases and a polyketide synthase Chem Biol. 7:623-42.
4. Konz *et al*. 1999. How do peptide synthetases generate structural diversity? Chem Biol. 6:R39-48.
5. Marahiel *et al*. 1997. Modular peptide synthetases involved in non-ribosomal peptide synthesis Chem. Rev. 97:2651-2673.
6. Schneider *et al*. 1998. Targeted alteration of the substrate specificity of peptide synthetases by rational module swapping Mol Gen Genet. 257:308-18.
7. Stachelhaus *et al*. 1995. Modular structure of peptide synthetases revealed by dissection of the multifunctional enzyme GrsA J Biol Chem. 270:6163-9.
8. Stachelhaus *et al*. 1999. The specificity-conferring code of adenylation domains in nonribosomal peptide synthetases Chem Biol. 6:493-505.
9. Stachelhaus *et al*. 1995. Rational design of peptide antibiotics by targeted replacement of bacterial and fungal domains Science. 269:69-72.

### EXAMPLE 7

### Oxazole derivatives

This example describes modulating the types of epothilone compounds produced by host cells using fermentation conditions. By supplementing host cells with excess serine, the compounds normally produced by host cells are modulated in such a way to favor the production of the oxazole counterparts. For example, cells that predominantly produce a compound or compounds of formula V can be made to favor the production of oxazole counterparts corresponding to compounds of formula VI. 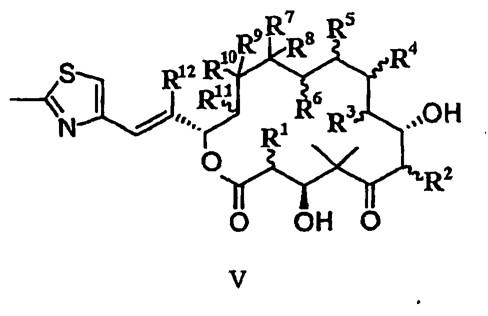 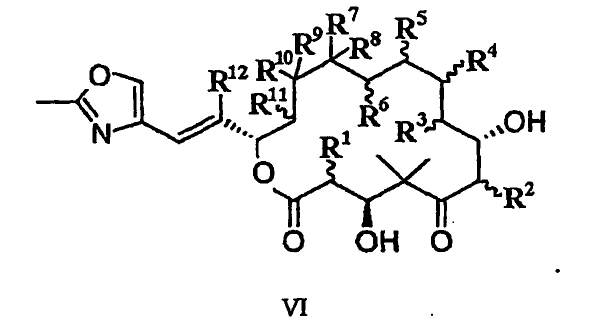 Wherein:
R¹, R², R³, R⁵, R¹¹, and R¹² are each independently hydrogen, methyl or ethyl;
R⁴, R⁶ and R⁹ are each independently hydrogen, hydroxyl, or oxo; alternatively
R⁵ and R⁶ together form a carbon carbon double bond;
R⁷ is hydrogen, methyl, or ethyl;
R⁸ and R¹⁰ are both hydrogen or together form a carbon carbon double bond or an epoxide;
Ar is aryl; and,
W is O or NR¹³ where R¹³ is hydrogen, C₁-C₁₀ aliphatic, aryl or alkylaryl. In another embodiment, compounds of formula I are provided wherein R¹, R², R³, R⁴, R⁵, R⁶, R⁷, R⁸, R⁹, R¹⁰, R¹¹, R¹², R¹³, Ar and W are as described previously provided that at least one of R¹, R⁴, R⁵, R⁶, R⁹ and R¹¹ is not hydrogen.

In one embodiment, *M. xanthus* strain K111-40-1, a strain that predominantly makes epothilones C and D is made to significantly increase the production of epothilones H₁ and H₂, the oxazole counterparts to epothilones C and D. Strain K111-40-1 (PTA-2712) was deposited in the American Type Culture Collection ("ATCC"), 10801 University Blvd., Manassas, VA, 20110-2209 USA on November 21, 2000. Strain K111-40-1 was grown in medium that was either supplemented or not supplemented with additional serine. The final concentrations of the components in unsupplemented medium were: hydrolyzed casein (pancreatic digest, purchased from Difco under the brand name Casitone), 5 g/L; MgSO4 ·7H₂O, 2 g/L; XAD-16, 20 g/L; trace elements solution 4 mL/L; methyl oleate 7 ml/L; and Hepes buffer, 40 mM (titrated to a pH of 7.6 with KOH). Trace elements solution comprises: concentrated H₂S0₄, 10 mL/L; FeCl₃·6H₂0, 14.6 g/L; ZnCl₂, 2.0 g/L; MnCl₂·4H₂O, 1.0 g/L; CuCl₂·2H₂O, 0.42 g/L; H₃BO₃, 0.31 g/L; CaCl₂.6H₂O, 0.24 g/L; and Na₂MoO₄·2H₂O, 0.24 g/L. The basal level of serine was taken as 4.82% w/w, the value determined by Difco' amino acid analysis of the particular lot of Casitone. Consequently, the basal serine concentration was 2.3 mM, a value calculated from the final concentration of 5 g/L of Casitone in the medium. Serine supplemented medium contained a fifty fold higher concentration of serine, 117 mM.

Cells were grown in flasks at 30 °C for 120 hours on a coffin shaker at 250 rpm. Compounds produced by the strains during fermentation were extracted by capturing the resin, washing the resin once in water, and the extracting the compounds in the resin for 30 minutes in 20 mL of methanol. The samples were analyzed on HPLC and by mass spectroscopy.

Analysis of the compounds produced by cells showed that a fifty fold increase in serine levels resulted in a 30 fold increase in the production of epothilone H₁ (0.12 mg/L) and a 5 fold increase in the production of epothilone H₂ (0.12 mg/L) over that produced by cells grown in medium that was not supplemented with serine. Notably, the cells produced almost undetectable quantities of epothilones C and D (< 50 µg/L).

The concomitant increase in oxazole-containing compounds and the decrease in thiazole-containing compounds from serine feeding provides a way to obtain the oxazole compounds from host cells that normally would make the thiazole-containing counterparts. For example, the recombinant construct to make 9-oxo epothilone D can be grown in conditions similar to that described above to make 17-des(2-methyl-4-thiazolyl)-17-(2-methyl-4-oxazolyl)-9-oxo-epothilone D, the oxazole-counterpart to 9-oxo-epothilone D. Similarly, other recombinant constructs of the invention including those described by Example 6 can be grown with excess serine to provide the corresponding oxazole compounds.

## Claims

1. A recombinant host cell of the suborder *Cystobacterineae* containing a recombinant expression vector that encodes an epothilone polyketide synthase (PKS) gene and produces a polyketide synthesized by a PKS enzyme encoded on said vector, wherein said cell is *Myxococcus xanthus* K111-40.1 deposited under ATCC accession no. PTA-2712, *Myxococcus xanthus* K111-72.4.4 deposited under ATCC accession No. PTA-2713 or *Myxococcus xanthus* strain K90-132.1.1.2 deposited under ATCC accession No. PTA-2715.

2. The cell of claim 1 that is *Myxococcus xanthus* K111-40.1 deposited under ATCC accession No. PTA-2712.

3. The cell of claim 1 that is *Myxococcus xanthus* K111-72.4.4 deposited under ATCC accession No. PTA-2713.

4. The cell of claim 1 that is *Myxococcus xanthus* strain K90-132.1.1.2 deposited under ATCC accession No. PTA-2715.

5. A method for producing an epothilone comprising culturing a host cell of any one of claims 1 to 4 under conditions such that said epothilone PKS gene encoded on said vector is expressed and an epothilone is produced.

6. The method of claim 5 wherein epothilone D is produced.

## Patentansprüche

1. Rekombinante Wirtszelle der Unterordnung *Cystobacterineae,* enthaltend einen rekombinanten Expressionsvektor, der ein Epothilon-Polyketidsynthase-(PKS)-Gen codiert und ein Polyketid produziert, synthetisiert durch ein PKS-Enzym, das auf diesem Vektor codiert ist, wobei die Zelle *Myxococcus xanthus* K111-40.1, hinterlegt unter der ATCC-Zugriffsnr. PTA-2712, *Myxococcus xanthus* K111-72.4.4, hinterlegt unter der ATCC-ZugrifFsnr. PTA-2713 oder *Myxococcus xanthus-Stamm* K90-132.1.1.2, hinterlegt unter der ATCC-Zugriffsnr. PTA-2715, ist.

2. Zelle nach Anspruch 1, die *Myxococcus xanthus* K111-40.1, hinterlegt unter der ATCC-Zugriffsnr. PTA-2712, ist.

3. Zelle nach Anspruch 1, die *Myxococcus xanthus* K111-72.4.4, hinterlegt unter der ATCC-Zugriffsnr. PTA-2713, ist.

4. Zelle nach Anspruch 1, die *Myxococcus xanthus-*Stamm K90-132.1.1.2, hinterlegt unter der ATCC-Zugriffsnr. PTA-2715, ist.

5. Verfahren zur Produktion eines Epothilons, umfassend das Züchten einer Wirtszelle nach einem der Ansprüche 1 bis 4 unter solchen Bedingungen, dass das Epothilon-PKS-Gen, das auf diesem Vektor codiert ist, exprimiert wird und ein Epothilon produziert wird.

6. Verfahren nach Anspruch 5, wobei Epothilon D produziert wird.

## Revendications

1. Cellule hôte recombinée appartenant au sous-ordre *Cystobacterineae* contenant un vecteur d'expression recombiné qui code pour un gène de l'épothilone polycétide synthase (PKS) et produit un polycétide synthétisé par une enzyme PKS codée sur ledit vecteur, dans laquelle ladite cellule est *Myxococcus xanthus* K111-40.1 déposée sous le numéro d'accession de l'ATCC PTA-2712, *Myxococcus xanthus* K111-72.4.4 déposée sous le numéro d'accession de l'ATCC PTA-2713 ou la souche *Myxococcus xanthus* K90-132.1.1.2 déposée sous le numéro d'accession de l'ATCC PTA-2715.

2. Cellule selon la revendication 1, qui est *Myxococcus xanthus* K111-40.1 déposée sous le numéro d'accession de l'ATCC PTA-2712.

3. Cellule selon la revendication 1, qui est *Myxococcus xanthus* K111-72.4.4 déposée sous le numéro d'accession de l'ATCC PTA-2713.

4. Cellule selon la revendication 1, qui est la souche *Myxococcus xanthus* K90-132.1.1.2 déposée sous le numéro d'accession de l'ATCC PTA-2715.

5. Procédé pour produire une épothilone comprenant la culture d'une cellule hôte selon l'une quelconque des revendications 1 à 4, dans des conditions telles que ledit gène de l'épothilone PKS codé sur ledit vecteur est exprimé et qu'une épothilone est produite.

6. Procédé selon la revendication 5, dans lequel l'épothilone D est produite.
